# DEMANDE DE BREVET EUROPEEN

(11) **EP 1 769 784 A1**
(43) Date de publication de la demande: **04.04.2007**
(21) Numéro de dépôt: 06120056.4
(22) Date de dépôt: 12.06.2002
(51) Int. Cl.: A61K 8/02, A61K 8/19, A61K 8/891, A61K 8/895, A61K 8/898, A61Q 1/02, A61Q 1/06

(54) **Composition à base d'huile siliconee structuree sous forme rigide, notamment pour une utilisation cosmetique**

(30) Priorité: 14.06.2001 FR 0107777
(62) Demande divisionnaire de: 02291467.5
(71) Demandeur: L'ORÉAL, 75008 Paris (FR)
(72) Inventeur: Ferrari, Véronique, 94700, MAISONS-ALFORT (FR); Mondet, Jean, 93600, AULNAY SOUS BOIS (FR)
(74) Mandataire: Poulin, Gérard

(57) **Abrégé**

L'invention concerne une composition physiologiquement acceptable, notamment cosmétique, comprenant une phase grasse liquide comprenant au moins une huile siliconée, structurée par un polymère de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène,

le polymère étant solide à la température ambiante et soluble dans la phase grasse liquide à une température de 25 à 250°C, des particules solides, et au moins une silicone amphiphile.

## Description

### Domaine technique

La présente invention se rapporte à une composition de soin et/ou de traitement et/ou de maquillage de la peau, y compris du cuir chevelu, et/ou des lèvres des êtres humains, contenant une phase grasse liquide comprenant au moins une huile siliconée, gélifiée par un polymère particulier, se présentant notamment sous forme d'un produit coulé de maquillage et en particulier d'un stick de maquillage comme les rouges à lèvres, dont l'application conduit à un dépôt brillant et non-migrant.

Elle concerne plus particulièrement des compositions cosmétiques et dermatologiques telles que des produits de maquillage, présentant des propriétés de tenue, de non-transfert et de stabilité.

### Etat de la technique antérieure

Dans les produits cosmétiques ou dermatologiques, il est courant de trouver une phase grasse liquide structurée, à savoir gélifiée et/ou rigidifiée ; ceci est notamment le cas dans les compositions solides comme les déodorants, les baumes et les rouges à lèvres, les fards à paupière, les produits anti-cerne et les fonds de teint coulés. Cette structuration est obtenue à l'aide de cires ou de charges. Malheureusement, ces cires et charges ont tendance à matifier la composition, ce qui n'est pas toujours souhaitable en particulier pour un rouge à lèvres.

Par phase grasse liquide, au sens de la demande, on entend une phase grasse liquide à température ambiante (25°C) et pression atmosphérique (760 mm de Hg), composée d'un ou plusieurs corps gras liquides à température ambiante, appelés aussi huiles, compatibles entre eux et contenant une huile siliconée.

Par phase grasse liquide structurée, au sens de la demande, on entend que cette phase structurée ne s'écoule pas entre les doigts et est au moins épaissie.

La structuration de la phase grasse liquide permet en particulier de limiter son exsudation des compositions solides et, en plus, de limiter, après dépôt sur la peau ou les lèvres, la migration de cette phase dans les rides et ridules, ce qui est particulièrement recherché pour un rouge à lèvres ou un fard à paupières. En effet, une migration importante de la phase grasse liquide, chargée de matières colorantes, conduit à un effet inesthétique autour des lèvres ou des yeux, accentuant particulièrement les rides et les ridules. Cette migration est souvent citée par les femmes comme un défaut majeur des rouges à lèvres classiques mais aussi des fards à paupières. Par migration, on entend un débordement de la composition déposée sur les lèvres ou la peau en dehors de son tracé initial.

La brillance est liée pour l'essentiel à la nature de la phase grasse liquide. Ainsi, il est possible de diminuer le taux de cires et de charges de la composition pour augmenter la brillance d'un rouge à lèvres, mais alors, la migration de la phase grasse liquide augmente. Autrement dit, les taux de cires et de charges nécessaires à la réalisation d'un stick de dureté convenable sont un frein à la brillance du dépôt.

Le document EP-A-1 068 856 [1] décrit des compositions cosmétiques solides, sans cire, comportant une phase grasse liquide structurée par un polymère, dans lesquelles la phase grasse est principalement une huile non siliconée.

L'utilisation de phases grasses à base d'huiles siliconées permet à ce jour d'obtenir des compositions cosmétiques ayant une longue tenue lorsque les huiles sont peu ou pas volatiles, à savoir une bonne tenue dans le temps, notamment de la couleur (non virage, non palissement), et des compositions non-transfert lorsque les huiles siliconées sont volatiles, à savoir ne se déposant pas sur un support tel qu'un verre, une tasse, un tissu ou une cigarette, placé au contact du film de maquillage.

Actuellement, l'utilisation d'huiles siliconées en cosmétique est limitée par le peu de molécules pouvant gélifier ces milieux et ainsi conduire à des compositions se présentant sous forme solide comme les bâtons de rouge à lèvres ou les fonds de teint coulés par exemple. La mise en oeuvre de compositions cosmétiques dont la phase grasse est majoritairement siliconée conduit dans la plupart des cas à des problèmes de compatibilité avec les ingrédients classiquement utilisés en cosmétique.

Dans les documents US-A-5 874 069 [2], US-A-5 919 441 [3], US-A-6 051 216 [4], WO-A-02/17870 [8] et WO-A-02/17871 [9],on a réalisé des compositions cosmétiques telles que des stick ou des gels de déodorant, comprenant une phase huileuse siliconée gélifiée par une cire à base de polysiloxane et polyamide, ou par un polymère contenant des groupes siloxane et des groupes capables d'interactions hydrogène.

Pour cette utilisation comme déodorant, les problèmes de migration de la phase huileuse dans les rides et ridules, ainsi que de tenue et de non-transfert de la composition ne se posent pas comme dans le cas des produits cosmétiques de maquillage décrits ci-dessus.

### Exposé de l'invention

L'invention a justement pour objet une composition de soin et/ou de maquillage et/ou de traitement de la peau et/ou des lèvres, permettant de remédier à ces inconvénients.

De façon surprenante, le demandeur a trouvé que l'utilisation de polymères particuliers en association avec des particules solides et au moins une silicone amphiphile permettait de structurer, en l'absence ou en présence de faibles quantités de cire, les phases grasses liquides à base d'huile siliconée sous forme de produit de maquillage ou de soin dont l'application conduisait à un film brillant ou mat et non migrant, d'améliorer l'homogénéité des compositions et de renforcer les propriétés de tenue et/ou de non-transfert de ces produits. En outre, leur stabilité thermique est améliorée.

L'invention s'applique non seulement aux produits de maquillage des lèvres comme les rouges à lèvres, les crayons à lèvres, les brillants à lèvres, mais aussi aux produits de soin et/ou de traitement de la peau, y compris du cuir chevelu, et des lèvres, comme les produits en stick de protection solaire de la peau, du visage ou des lèvres, ou les baumes à lèvres, aux produits de maquillage de la peau, aussi bien du visage que du corps humain, comme les fonds de teints coulés en stick ou en coupelle, les produits anti-cerne et les produits de tatouage éphémère, aux produits d'hygiène et de nettoyage, notamment en stick, et aux produits de maquillage des yeux comme les eye-liners en particulier sous forme de crayon et les mascaras notamment pains pour fibres kératiniques (cils, sourcils, cheveux).

De façon plus précise, l'invention a pour objet une composition comprenant une phase grasse liquide comprenant au moins une huile siliconée, structurée par l'association :
1) d'au moins un agent gélifiant constitué par un polymère (homopolymère ou copolymère) de masse moléculaire moyenne en poids allant de 500 à 500 000, comportant au moins un motif comprenant :
   - au moins un groupe polyorganosiloxane, constitué de 1 à 1000 unités organosiloxanes dans la chaîne du motif ou sous forme de greffon, et
   - au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
   le polymère étant solide à la température ambiante et soluble dans la phase grasse liquide à une température de 25 à 250°C,
2) de particules solides, et
3) d'au moins une silicone amphiphile,
la phase grasse liquide, le polymère, les particules solides et la silicone amphiphile formant un milieu physiologiquement acceptable.

Selon l'invention, la composition peut être sous forme d'un solide déformable ou non.

Selon l'invention, les particules solides utilisées dans les compositions peuvent être des charges ou des pigments. Généralement la taille moyenne des particules solides est de 10 nm à 50 µm, de préférence de 50 nm à 30 µm et mieux encore de 100 nm à 10 µm. Elles peuvent être sous forme de poudres, de fibres ou de plaquettes.

Ces charges utilisées dans les compositions cosmétiques ont généralement pour but d'absorber la sueur et le sébum ou d'apporter de la matité. Selon l'invention, elles permettent de plus de structurer la phase grasse liquide comportant une huile siliconée et de renforcer les propriétés de tenue et/ou de non-transfert de la composition ainsi que la stabilité thermique.

Dans une composition anhydre en stick comme dans les rouges à lèvres et les produits anti-cernes en bâton, les charges permettent en outre, de limiter l'exsudation de l'huile en dehors du bâton même à chaud (45-47°C) et/ou de limiter la migration de la phase grasse liquide, en dehors de son tracé original, notamment dans les rides et ridules.

Par pigments, on entend toute particule solide insoluble dans la composition servant à donner et/ou modifier une couleur et/ou aspect irisé.

Ces pigments peuvent à la fois assurer la fonction d'absorption de la sueur et du sébum, et la fonction de coloration ou de modification d'aspect de la composition, soit du produit cosmétique de maquillage, de traitement ou d'hygiène corporelle. Dans l'invention, ils assurent également la structuration de la phase grasse liquide.

Ces charges ou pigments peuvent être soit de nature hydrophobe, soit de nature hydrophile. De préférence, l'invention s'applique à des particules hydrophiles qui sont plus difficiles à disperser dans des milieux très siliconés.

En effet, selon l'invention, on facilite la dispersion de telles particules hydrophiles au moyen d'au moins une silicone amphiphile qui joue le rôle de tensioactif entre les particules hydrophiles et la phase siliconée hydrophobe.

Ces silicones amphiphiles comportent une partie silicone qui est compatible avec le milieu très siliconé des compositions de l'invention, et une partie hydrophile qui peut être, par exemple, le reste d'un composé choisi parmi les alcools et les polyols, ayant de 1 à 12 groupements hydroxyle, les polyoxyalkylènes comportant au moins deux motifs oxyalkylénés et ayant de 0 à 20 motifs oxypropylénés et/ou de 0 à 20 motifs oxyéthylénés. Cette partie hydrophile a donc une affinité pour les particules hydrophiles et favorise leur dispersion dans le milieu siliconé.

Ces particules hydrophiles, sous forme de poudres ou de fibres, peuvent être constituées par des pigments et/ou des nacres permettant d'obtenir un maquillage couvrant, c'est-à-dire ne laissant pas voir la peau, les lèvres ou les phanères. Ces particules permettent en outre de réduire le toucher collant des compositions.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés (ou nacres) peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

De préférence, dans l'invention, on utilise des particules hydrophiles constituées par des pigments choisis parmi les oxydes de titane, les oxydes de zinc et les oxydes de fer, en particulier ceux dont la taille élémentaire des particules est inférieure à 1 µm ; ces oxydes sont appelés nanooxydes ou nanopigments.

On peut aussi utiliser dans l'invention d'autres charges et/ou pigments hydrophiles tels que la silice, l'alumine, les talcs, les argiles, le carbonate de calcium et l'amidon.

Selon l'invention, la composition peut aussi comprendre des pigments et/ou des charges hydrophobes constitués par des poudres de polymères ou copolymères hydrophobes réticulés. A titre d'exemple de polymères et copolymères hydrophobes réticulés, on peut citer :
1°) les polymères fluorés tels que les poudres de polytétrafluoroéthylène et les poudres de copolymère de tétrafluoroéthylène et d'oléfine, par exemple d'éthylène ou de propylène ;
2°) les élastomères silicones, par exemples des poudres de polyméthylsilsesquioxane ;
3°) les polyoléfines telles que le polyéthylène ;
4°) les polyméthacrylates d'alkyle, par exemple le polyméthacrylate de méthyle ;
5°) les polyamides ;
6°) les polystyrènes et dérivés, par exemple le polyméthylstyrène ;
7°) les polyesters ;
8°) les polyacryliques ; et
9°) les polyuréthanes, par exemple les poudres d'hexaméthylène diisocyanate (HDI)/triméthylol hexalactone.

Au lieu de poudres, on peut bien entendu utiliser des charges ou pigments sous forme de fibres, ainsi que sous forme de plaquettes.

D'autres particules hydrophobes peuvent être constituées par des particules de lauroyllysine.

Comme on l'a vu précédemment, la composition de l'invention comprend une silicone amphiphile permettant d'obtenir une dispersion homogène des particules solides hydrophiles (charges et/ou pigments) dans le milieu siliconé.

Cette silicone amphiphile peut être une huile sans activité gélifiante. De telles huiles peuvent être constituées par :
- des diméthicone copolyols, comportant éventuellement des groupes phényle,
- des alkylméthicone copolyols,
- des silicones polyglycérolées, c'est-à-dire des silicones à groupes alkylglycéryl éthers,
- des silicones à groupes latéraux perfluorés et à groupes latéraux glycérolés,
- des silicones à groupes latéraux polyoxyéthylénés/polyoxypropylénés et à groupes latéraux perfluorés,
- des copolymères à bloc silicone et à bloc hydrophile autre que polyéther, par exemple polyoxazoline ou polyéthylèneimine,
- des copolymères greffés du type polysaccharides greffés silicone,
- des copolymères à bloc silicone à bloc poly(oxyde d'éthylène/oxyde de propylène).

La silicone amphiphile utilisée dans l'invention peut être aussi une résine silicone amphiphile au moins partiellement réticulée.

A titre d'exemple de telles résines, on peut citer :
- les résines silicones réticulées à groupes alkylpolyéther, tels que polyoxyde d'éthylène (POE) et polyoxyde d'éthylène/polyoxyde de propylène (POE/POP), décrites dans US-A-5 412 004 [6], et
- les résines silicones réticulées partiellement par des α,ω-diènes, possédant à la fois des chaînes latérales POE/POP hydrophiles et des chaînes latérales alkyle hydrophobes telles que celles décrites dans EP-A-1 048 686 [7]. Les chaînes latérales hydrophiles sont obtenues par réaction avec un POE/POP à une seule extrémité vinylique, et les chaînes latérales alkyle sont formées par réaction avec une α-oléfine à chaîne grasse.

Dans la résine silicone amphiphile, la partie silicone est avantageusement formée de polydiméthylsiloxane.

Dans la composition de l'invention, le polymère jouant le rôle d'agent gélifiant, représente généralement de 0,5 à 80% du poids total de la composition, de préférence de 2% à 60% et mieux encore de 5 à 40%, les particules solides représentent généralement de 0,1 à 90%, de préférence de 1 à 70% et mieux de 2 à 50%, par exemple de 5 à 25% du poids total de la composition, et la silicone amphiphile représente généralement de 0,1 à 20%, de préférence de 0,1 à 10%, du poids total de la composition, le reste étant constitué par la phase grasse liquide et d'autres additifs éventuels.

Par ailleurs, le rapport massique polymère gélifiant/huile(s) siliconée(s) est de préférence de 0,1 à 50%.

Selon l'invention, on peut aussi utiliser des particules solides hydrophiles qui ont été soumises à un traitement hydrophobe.

Il peut s'agir d'un enrobage ou d'un greffage par un composé hydrophobe.

L'enrobage peut consister en un traitement de surface des particules avant leur introduction dans la phase grasse, par exemple lors de leur fabrication, ou in situ.

L'enrobage ou traitement de surface peut être un enrobage fluoré tel qu'un mono- ou diester perfluoroalkyle d'acide phosphorique, (acide ou sel), un perfluoropolyéther, un perfluoroacide carboxylique ou sulfonique, ou un sel de perfluoroalkyl phosphate de diéthanolamine.

L'enrobage peut être un enrobage ou un greffage à base de silicone fluorée, par exemple un greffage par un silane à groupe perfluoroalkyle.

Le traitement de surface peut aussi être effectué au moyen de dérivés siliconés, par exemple un greffage par des silicones réactives possédant initialement des groupes hydrogénosilanes, un greffage par un diorganosilane tel que le diméthylchlorosilane ou par un alkylalcoxysilane, un greffage par un silane à groupe glycydoxypropyle, un enrobage par une silicone polyglycérolée, ou un enrobage par un copolymère acrylique greffé silicone ou silicone-g-polyacrylique.

On peut encore utiliser un enrobage par des N-acylaminoacides, par exemple la N-lauroyllysine, des enrobages par des acides gras ou sels d'acide gras du type acide stéarique, des enrobages par des lécithines et des enrobages par des huiles esters.

On peut aussi ajouter un dispersant à la composition de l'invention.

La phase grasse liquide contient avantageusement au moins 40% et mieux encore au moins 50% en poids d'huile (s) siliconée (s), ayant avantageusement une viscosité inférieure à 5 000 cSt et mieux encore inférieure à 3 000 cSt car les polymères siliconés utilisés dans l'invention sont plus solubles dans les huiles siliconées de faible viscosité. Elle peut contenir également d'autres huiles ou mélange d'huiles non siliconées.

Les huiles siliconées utilisables dans l'invention peuvent être en particulier les polydiméthylsiloxanes (PDMS) volatils ou non, linéaires ou cycliques, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, des diphényl diméthicones, les diphényl méthyldiphényl trisiloxanes, et les 2-phényléthyl triméthylsiloxysilicates.

La phase grasse liquide peut aussi contenir d'autres huiles non siliconées, par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel ;
- les huiles ou esters de synthèse de formule R₅COOR₆ dans laquelle R₅ représente le reste d'un acide gras supérieure linéaire ou ramifié comportant de 1 à 40 et mieux de 7 à 19 atomes de carbone et R₆ représente une chaîne hydrocarbonée ramifiée contenant de 1 à 40 et mieux de 3 à 20 atomes de carbone, avec R₅ + R₆ > 10 comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl-2-hexyle, des octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₆ comme l'alcool oléique ; et
- leurs mélanges.

La phase grasse liquide peut encore contenir des huiles apolaires telles que les hydrocarbures ou fluorocarbures, linéaires ou ramifiés, d'origine synthétique ou minérale, volatils ou non, comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges.

Généralement, la phase grasse liquide représente de 5 à 99% du poids total de la composition et mieux de 20 à 75%.

Les polymères utilisés comme agents gélifiants dans la composition de l'invention sont des polymères du type polyorganosiloxane tels que ceux décrits dans les documents US-A-5 874 069 [2], US-A-5, 919, 441 [3], US-A-6,051,216 [4] et US-A-5,981,680 [5].

Selon l'invention, les polymères utilisés comme agent gélifiant peuvent appartenir aux deux familles suivantes :
1) des polyorganosiloxanes comportant au moins deux groupes capables d'établir des interactions hydrogène, ces deux groupes étant situés dans la chaîne du polymère ; et/ou
2) des polyorganosiloxanes comportant au moins deux groupes capables d'établir des interactions hydrogène, ces deux groupes étant situés sur des greffons ou ramifications.

Les polymères auxquels s'applique l'invention, sont des solides qui peuvent être solubilisés au préalable dans un solvant à interactions hydrogène, capable de rompre les interactions hydrogène des polymères comme les alcools inférieurs en C₂ à C₈ et notamment l'éthanol, le n-propanol, l'isopropanol, avant d'être mis en présence des huiles siliconées selon l'invention. Il est aussi possible d'utiliser ces solvants "rupteurs" d'interactions hydrogène comme co-solvant. Ces solvants peuvent ensuite être conservés dans la composition ou bien être éliminés par évaporation sélective, bien connue de l'homme de l'art.

Les polymères comportant deux groupes capables d'établir des interactions hydrogène dans la chaîne du polymère peuvent être des polymères comprenant au moins un motif répondant à la formule : 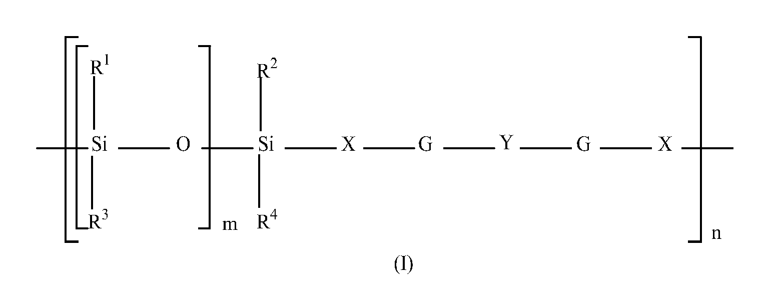 dans laquelle :
1) R¹, R², R³ et R⁴, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote ;
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote ;
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 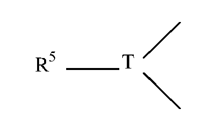 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R⁵ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié à une autre chaîne du polymère,
5) les G, identiques ou différents, représentent les groupes divalents choisis parmi : 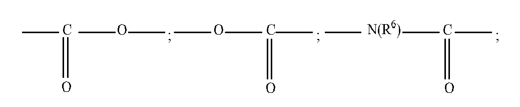 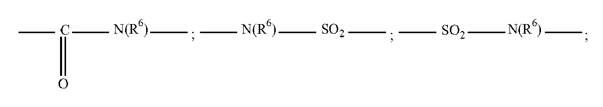 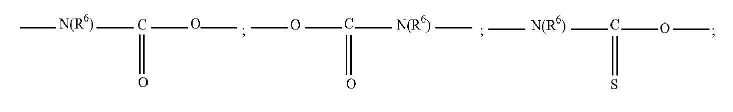 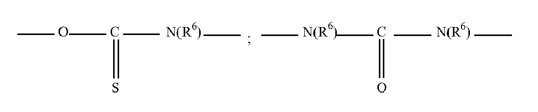 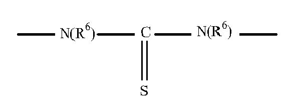 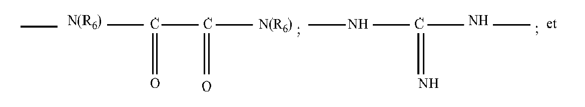 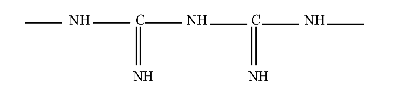 où R⁶ représente un atome d'hydrogène ou un groupe alkyle, linéaire ou ramifié, en C₁ à C₂₀, à condition qu'au moins 50% des R⁶ du polymère représente un atome d'hydrogène et qu'au moins deux des groupes G du polymère soient un autre groupe que : 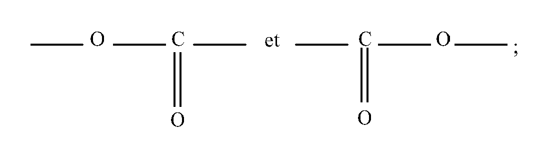
6) n est un nombre entier allant de 2 à 500, de préférence de 2 à 200, et m est un nombre entier allant de 1 à 1000, de préférence de 1 à 700 et mieux encore de 6 à 200.

Selon l'invention, 80% des R¹, R², R³ et R⁴, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆,
g) les chaînes polyorganosiloxane de formule : 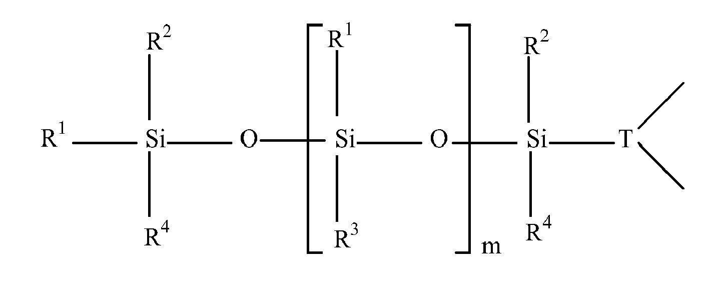 dans laquelle R¹, R², R³, R⁴, T et m sont tels que définis ci-dessus, et
h) les chaînes polyorganosiloxanes de formule :

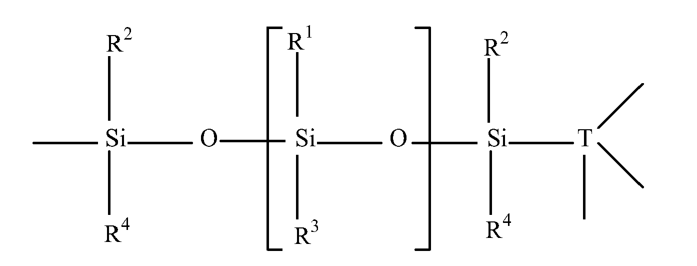

Les polyorganosiloxanes de la seconde famille peuvent être des polymères comprenant au moins un motif répondant à la formule (II) : 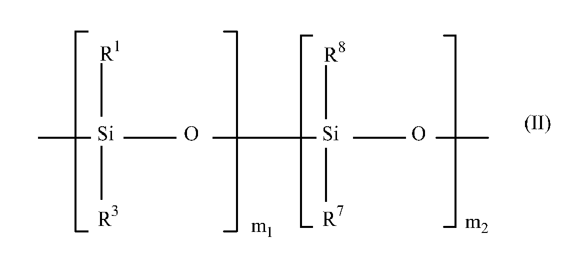 dans laquelle
- R¹ et R³, identiques ou différents, sont tels que définis ci-dessus pour la formule (I),
- R⁷ représente un groupe tel que défini ci-dessus pour R¹ et R³, ou représente le groupe de formule -X-G-R⁹ dans laquelle X et G sont tels que définis ci-dessus pour la formule (I) et R⁹ représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
- R⁸ représente le groupe de formule -X-G-R⁹ dans laquelle X, G et R⁹ sont tels que définis ci-dessus,
- m₁ est un nombre entier allant de 1 à 998, et
- m₂ est un nombre entier allant de 2 à 500.

Selon l'invention, le polymère utilisé comme agent gélifiant, peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R¹, R², R³, R⁴, X, G, Y, m et n est différent dans l'un des motifs. Le copolymère peut être aussi formé de plusieurs motifs de formule (II), dans lequel l'un au moins des R¹, R³, R⁷, R⁸, m₁ et m₂ est différent dans l'un au moins des motifs.

On peut encore utiliser un copolymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, thiourée et leurs combinaisons. Ces copolymères peuvent être des copolymères blocs, des copolymères séquencés ou des copolymères greffés.

Selon un premier mode de réalisation de l'invention, les groupes capables d'établir des interactions hydrogènes sont des groupes amides de formule -C(O)NH- et -HN-C(O)-.

Dans ce cas, l'agent gélifiant peut être un polymère comprenant au moins un motif de formule (III) ou (IV) : 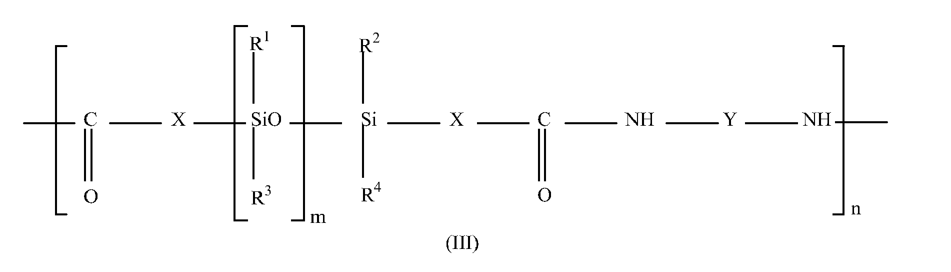 ou 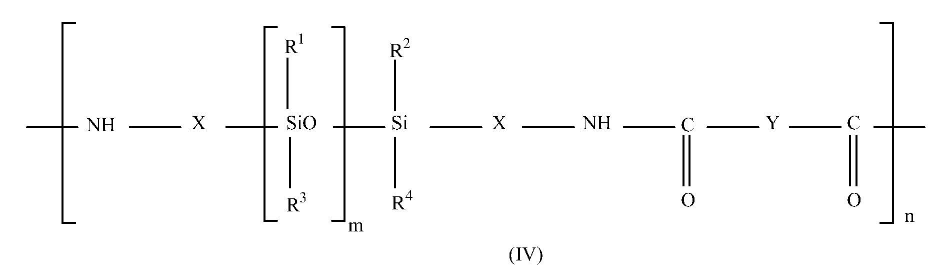 dans lesquelles R¹, R², R³, R⁴, X, Y, m et n sont tels que définis ci-dessus.

Un tel motif peut être obtenu :
- soit par une réaction de condensation entre un silicone à extrémités α, ω-acides carboxyliques et une
ou plusieurs diamines, selon le schéma réactionnel suivant : 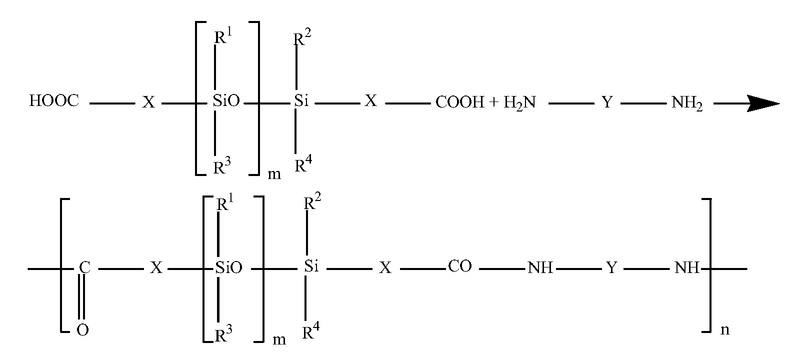
- soit par réaction de deux molécules d'acide carboxylique α-insaturé avec une diamine selon le schéma réactionnel suivant :

   CH₂=CH-X¹-COOH+H₂N-Y-NH₂ → CH₂=CH-X¹-CO-NH-Y-NH-CO-X¹-CH=CH₂

   suivie de l'addition d'un siloxane sur les insaturations éthyléniques, selon le schéma suivant :

   CH₂=CH-X¹-CO-NH-Y-NH-CO-X¹-CH=CH₂

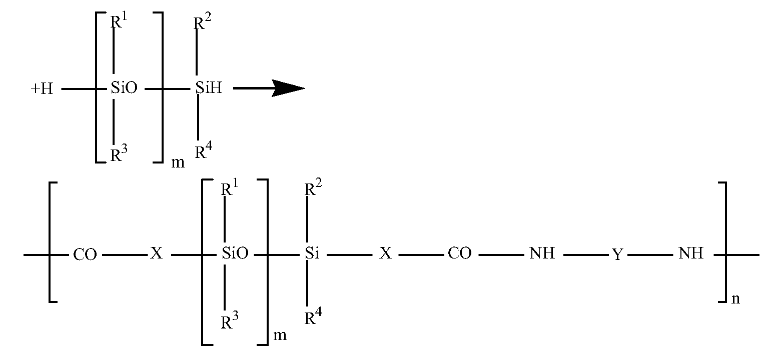

   dans lesquels X¹-(CH₂)₂- correspond au X défini ci-dessus et Y, R¹, R², R³, R⁴ et m sont tels que définis ci-dessus ;
- soit par réaction d'un silicone à extrémités α, ω-NH₂ et d'un diacide de formule HOOC-Y-COOH selon le schéma réactionnel suivant :

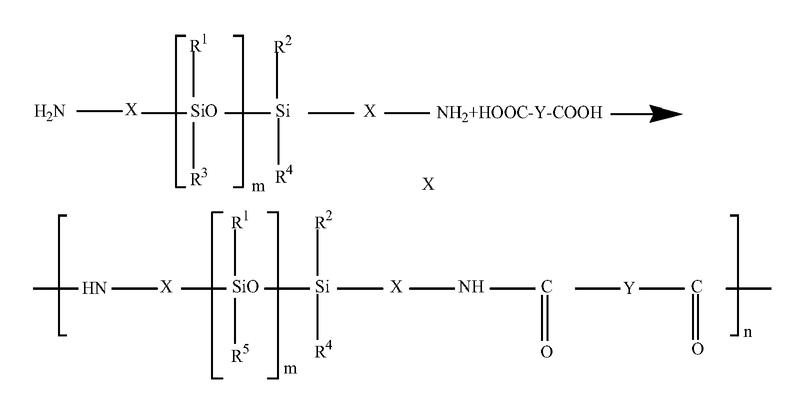

Dans ces polyamides de formule (III) ou (IV), m est de préférence dans la gamme de 1 à 700, de préférence de 15 à 500 et mieux encore de 15 à 45, et n est en particulier dans la gamme de 1 à 500, de préférence de 1 à 100 et mieux encore de 4 à 25,
X est de préférence une chaîne alkylène linéaire ou ramifiée ayant de 1 à 30 atomes de carbone, en particulier 3 à 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :
1°) 1 à 5 groupes amides, urée ou carbamate,
2°) un groupe cycloalkyle en C₅ ou C₆, et
3°) un groupe phénylène éventuellement substitué par 1 à 3 groupes alkyles identiques ou différents en C₁ à C₃.

Dans les formules (III) et (IV), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans ces formules (III) et (IV), Y peut aussi représenter : 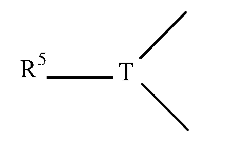 où R⁵ représente une chaîne polyorganosiloxane, et T représente un groupe de formule : 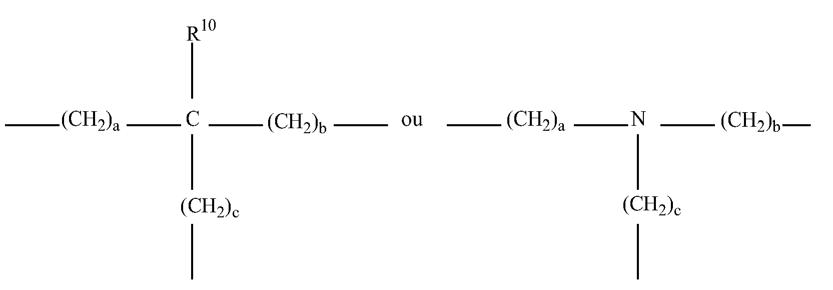 dans lesquelles a, b et c sont indépendamment des nombres entiers allant de 1 à 10, et R¹⁰ est un atome d'hydrogène ou un groupe tel que ceux définis pour R¹, R², R³ et R⁴.

Dans les formules (III) et (IV), R¹, R², R³ et R⁴ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule : 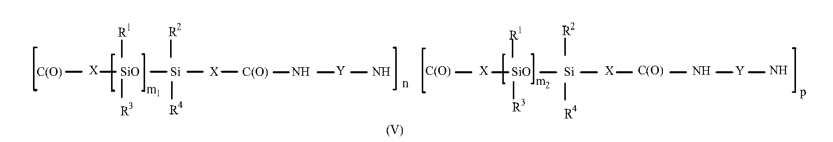 dans laquelle X, Y, n, R¹ à R⁴ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la formule : 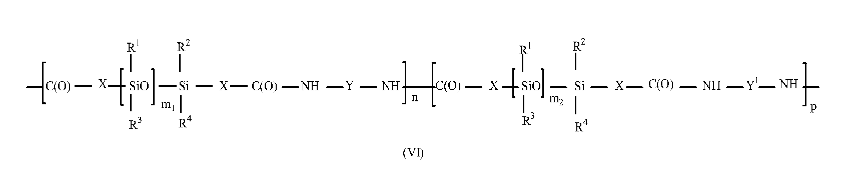 dans laquelle R¹ à R⁴, X, Y, m₁, m₂, n et p ont les significations données ci-dessus et Y¹ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, l'agent gélifiant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif de formule : 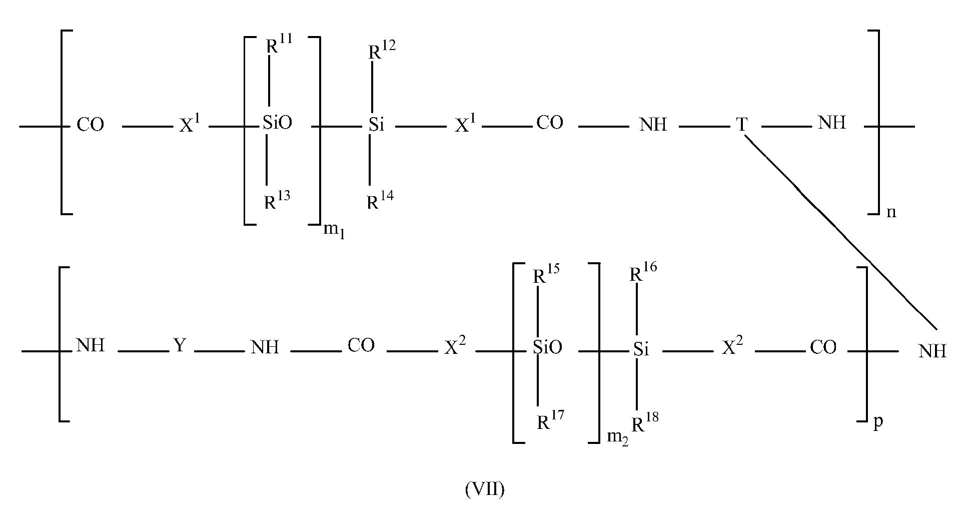 dans laquelle X¹ et X² qui sont identiques ou différents, ont la signification donnée pour X dans la formule (I), n est tel que défini dans la formule (I), Y et T sont tels que définis dans la formule (I), R¹¹ à R¹⁸ sont des groupes choisis dans le même groupe que les R¹ à R⁴, m₁ et m₂ sont des nombres situés dans la gamme allant de 1 à 1 000, et p est un nombre entier allant de 2 à 500.

Dans la formule (VII), on préfère que :
- p soit dans la gamme de 1 à 25, mieux encore de 1 à 7,
- R¹¹ à R¹⁸ soient des groupes méthyle,
- T réponde à l'une des formules suivantes : 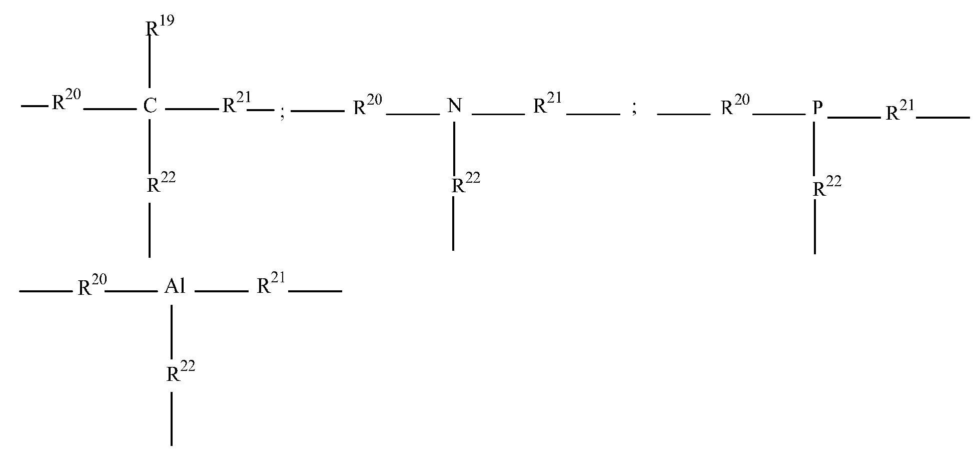 dans lesquelles R¹⁹ est un atome d'hydrogène ou un groupe choisi parmi les groupes définis pour R¹ à R⁴, et R²⁰, R²¹ et R²² sont indépendamment des groupes alkylène, linéaires ou ramifiés, de préférence encore, à la formule : 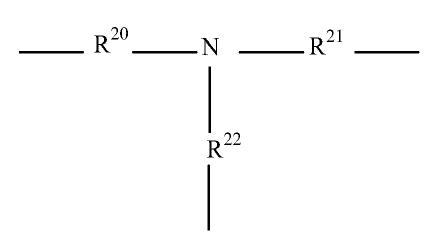 en particulier avec R²⁰, R²¹ et R²² représentant -CH₂-CH₂-,
- m₁ et m₂ soient dans la gamme de 15 à 500, et mieux encore de 15 à 45,
- X¹ et X² représentent -(CH₂)₁₀-, et
- Y représente -CH₂-.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids des motifs silicone greffé (VII) dans le copolymère peut aller de 0,5 à 30% en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon l'invention, les polyamides à base de siloxanes préférés sont :
- les polyamides de formule (III) où m est de 15 à 50 ;
- les mélanges de deux ou plusieurs polyamides dans lesquels au moins un polyamide a une valeur de m dans la gamme de 15 à 50 et au moins un polyamide a une valeur de m dans la gamme de 30 à 50 ;
- des polymères de formule (V) avec m₁ choisi dans la gamme de 15 à 50 et m₂ choisi dans la gamme de 30 à 500 avec la partie correspondant à m₁ représentant 1 à 99% en poids du poids total du polyamide et la partie correspondant à m₂ représentant 1 à 99% en poids du poids total du polyamide ;
- des mélanges de polyamide de formule (III) combinant
   1) 80 à 99% en poids d'un polyamide où n est égal à 2 à 10, en particulier 3 à 6, et
   2) 1 à 20% d'un polyamide où n est dans la gamme de 5 à 500, en particulier de 6 à 100 ;
- des polyamides répondant à la formule (VI) où au moins l'un des Y et Y¹ contient au moins un substituant hydroxyle.
- des polyamides de formule (III) synthétisés avec au moins une partie d'un diacide activé (chlorure, dianhydride ou diester de diacide) au lieu du diacide ;
- des polyamides de formule (III) où X représente -(CH₂)₃- ou -(CH₂)₁₀ ; et
- des polyamides de formule (III) où les polyamides sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Selon l'invention, les extrémités des chaînes du polymère peuvent être terminées par :
- un groupe ester d'alkyle en C₁ à C₅₀ en introduisant en cours de synthèse un monoalcool en C₁ en C₅₀,
- un groupe amide d'alkyle en C₁ à C₅₀ en prenant comme stoppeur un monoacide si la silicone est α, ω-diaminée, ou une monoamine si la silicone est α, ω-diacide carboxylique.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Des agents gélifiants à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamine et/ou des oligosiloxanes-diamines (réaction d'amidation), ou alternativement avec des oligosiloxanes alcools ou des oligosiloxanes diols (réaction d'estérification). La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonées, des siloxanes diamines avec 1 à 300, plus particulièrement 2 à 50, et mieux encore 2, 6, 9, 5, 12, 13,5, 23 ou 31 groupes siloxanes, peuvent être utilisés pour la réaction avec des polyamides hydrocarbonées à base de dimères d'acide gras. On préfère des siloxanes diamines ayant 13,5 groupes siloxanes et les meilleurs résultats sont obtenus avec la siloxane-diamine ayant 13,5 groupes siloxane et des polyamides contenant des indices élevés de groupes terminaux acides carboxyliques.

Les réactions peuvent être effectuées dans le xylène pour extraire l'eau produite de la solution par distillation azéotropique, ou à des températures plus élevées (autour de 180 à 200°C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1% en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

On peut aussi préparer un copolymère de polyamide-silicone, en utilisant un polyamide à groupes amines libres, par réaction d'amidation avec un siloxane contenant un groupe acide.

On peut encore préparer un agent gélifiant à base de copolymère entre un polyamide hydrocarboné et un polyamide siliconé, par transamidation d'un polyamide ayant par exemple un constituant éthylène-diamine, par une oligosiloxane-α, ω-diamine, à température élevée (par exemple 200 à 300°C), pour effectuer une transamidation de sorte que le composant éthylène diamine du polyamide d'origine est remplacé par l'oligosiloxane diamine.

Le copolymère de polyamide hydrocarboné et de polyamide-silicone peut encore être un copolymère greffé comportant un squelette de polyamide hydrocarboné avec des groupes oligosiloxane pendants.

Ceci peut être obtenu par exemple :
- par hydrosilylation de liaisons insaturées dans des polyamides à base de dimères d'acides gras ;
- par silylation des groupes amides d'un polyamide ; ou
- par silylation de polyamides insaturés au moyen d'une oxydation, c'est-à-dire en oxydant les groupes insaturés en alcools ou diols, pour former des groupes hydroxyle que l'on fait réagir avec des acides siloxane carboxyliques ou des siloxanes-alcools. On peut aussi époxyder les sites oléfiniques des polyamides insaturés puis faire réagir les groupes époxy avec des siloxanes-amines ou des siloxanes-alcools.

Selon un second mode de réalisation de l'invention, l'agent gélifiant est constitué par un homopolymère ou copolymère comportant des groupes uréthane ou urée.

Comme précédemment, le polymère peut comporter des motifs polyorganosiloxanes contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante : 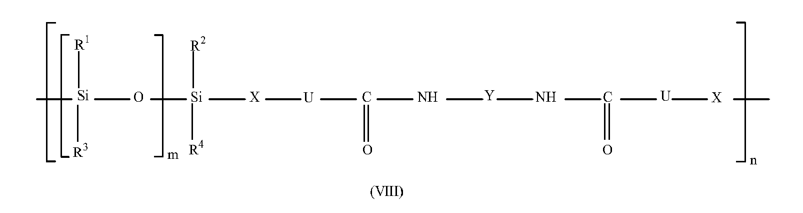 dans laquelle les R¹, R², R³, R⁴, X, Y, m et n ont les significations données ci-dessus pour la formule (I), et U représente -O- ou -NH-, afin que : 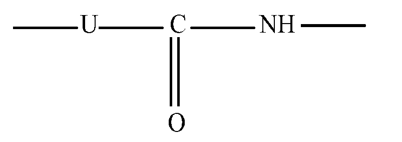 corresponde à un groupe uréthane ou urée.

Dans cette formule (VIII), Y peut être un groupe alkylène, en C₁ à C₄₀, linéaire ou ramifié, substitué éventuellement par un groupe alkyle en C₁ à C₁₅ ou un groupe aryle en C₅ à C₁₀. De préférence, on utilise un groupe -(CH₂)₆-.

Y peut aussi représenter un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en C₁ à C₄₀, linéaire ou ramifié, ou un radical cycloalkylène en C₄ à C₁₂.

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane ou urée dans la chaîne alkylène.

Il peut répondre à la formule : 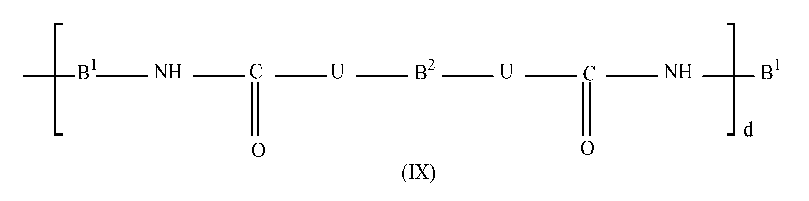 dans laquelle B¹ est un groupe choisi parmi les groupes donnés ci-dessus pour Y, U est -O- ou -NH-, et B² est choisi parmi :
- les groupes alkylène en C₁ à C₄₀, linéaires ou ramifiés, qui peuvent porter éventuellement un groupe ionisable tel qu'un groupe acide carboxylique ou sulfonique, ou un groupe amine tertiaire neutralisable ou quaternisable,
- les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en C₁ à C₃, et
- les groupes de formule : 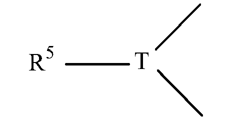 dans laquelle T est un radical trivalent hydrocarboné pouvant contenir un ou plusieurs hétéroatomes tels que l'oxygène, le soufre et l'azote et R⁵ est une chaîne polyorganosiloxane ou une chaîne alkyle en C₁ à C₅₀, linéaire ou ramifiée.

T peut représenter par exemple : 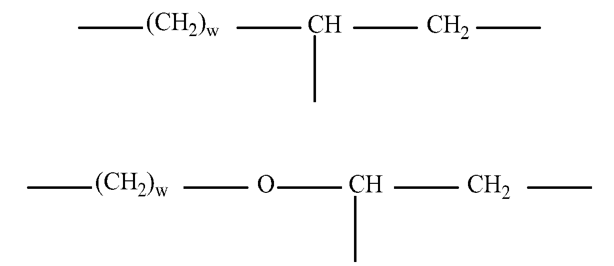 avec w étant un nombre entier allant de 1 à 10 et R⁵ étant une chaîne polyorganosiloxane.

Lorsque Y est un groupe alkylène, en C₁ en C₄₀ linéaire ou ramifié, on préfère les groupes -(CH₂)₂- et -(CH₂)₆-.

Dans la formule donnée ci-dessus pour Y, d peut être un entier allant de 0 à 5, de préférence de 0 à 3, de préférence encore égal à 1 ou 2.

De préférence B² est un groupe alkylène en C₁ à C₄₀, linéaire ou ramifié, en particulier -(CH₂)₂- ou-(CH₂)₆-, ou le groupe : 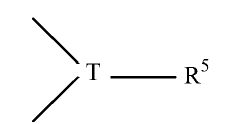 avec R⁵ étant une chaîne polyorganosiloxane.

Comme précédemment, le polymère constituant l'agent gélifiant peut être formé de motifs silicone uréthane et/ou silicone-urée de longueur et/ou de constitution différentes, et se présenter sous la forme de copolymères blocs, séquencés ou aléatoires.

Selon l'invention, le silicone peut aussi comporter les groupes uréthane et/ou urée non plus dans le squelette mais en ramifications latérales.

Dans ce cas, le polymère peut comprendre au moins un motif de formule : 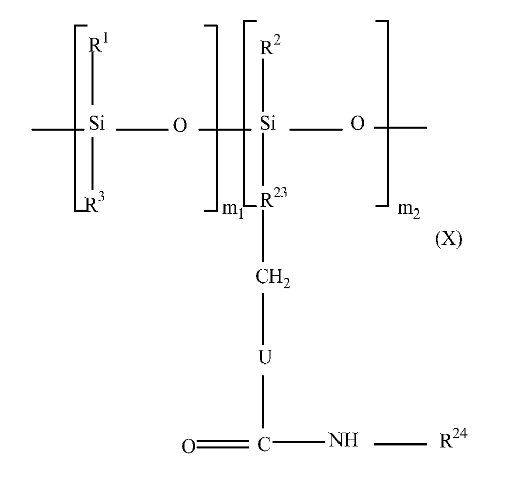 dans laquelle R¹, R², R³, m₁ et m₂ ont les significations données ci-dessus pour la formule (I),
- U représente O ou NH,
- R²³ représente un groupe alkylène en C₁ à C₄₀, comportant éventuellement un ou plusieurs hétéroatomes choisis parmi O et N, ou un groupe phénylène, et
- R²⁴ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

Les polymères comportant au moins un motif de formule (X) contiennent des unités siloxanes et des groupes urées ou uréthanes, et ils peuvent être utilisés comme agents gélifiants dans les compositions de l'invention.

Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.

Ils peuvent être obtenus à partir de polysiloxanes ramifiés, comportant un ou deux groupes amino par ramification, en faisant réagir ces polysiloxanes avec des monoisocyanates.

A titre d'exemples de polymères de départ de ce type ayant des ramifications amino et diamino, on peut citer les polymères répondant aux formules suivantes :

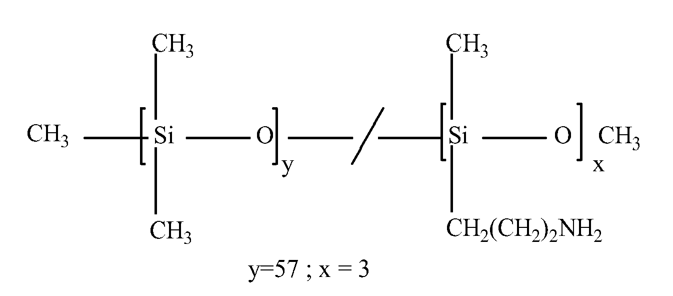

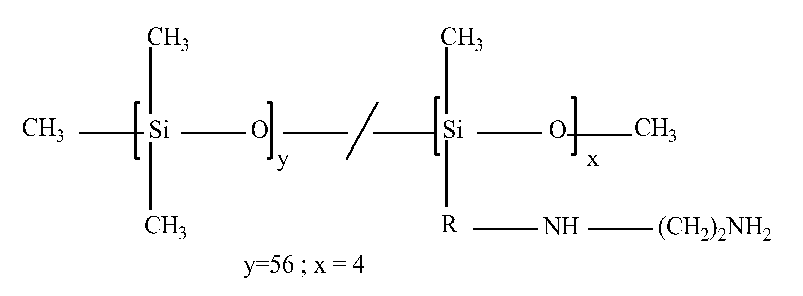

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.

De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales, ou bien les groupes amino peuvent être aux extrémités de la chaîne principale de sorte que les groupes capables d'interaction hydrogène seront des groupes terminaux du polymère.

Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.

Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.

A titre d'illustration d'un polysiloxane terminé par quatre groupes urées, on peut citer le polymère de formule : 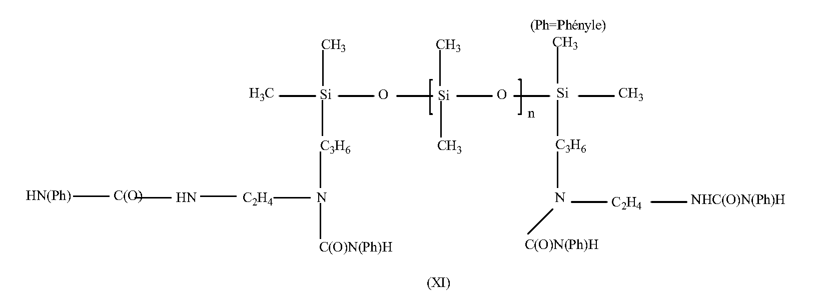 où Ph est un groupe phényle et n est un nombre de 0 à 300, en particulier de 0 à 100, par exemple de 50.

Ce polymère est obtenu par réaction du polysiloxane à groupes amino suivant : 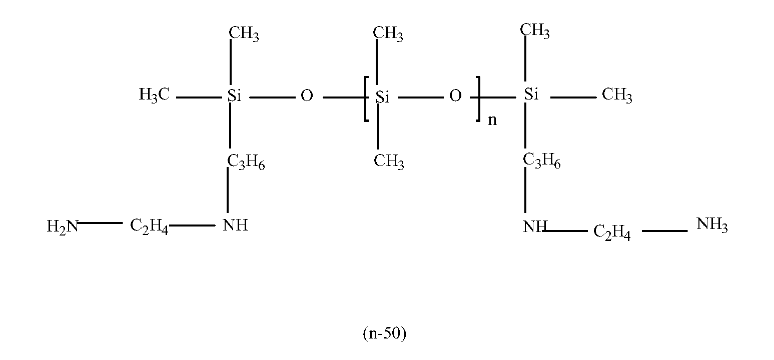 avec l'isocyanate de phényle.

Les polymères de formule (VIII) comportant des groupes urées ou uréthanes dans la chaîne du polymère siliconé peuvent être obtenus par réaction entre un silicone à groupes terminaux α,ω-NH₂ ou -OH, de formule : 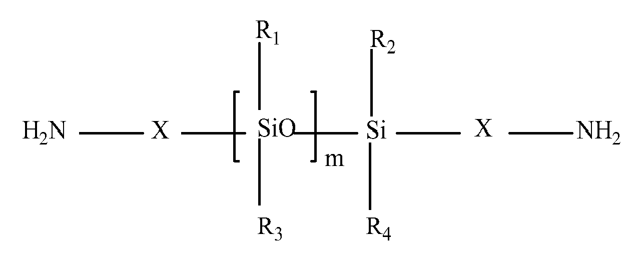 dans laquelle m, R¹, R², R³, R⁴ et X sont tels que définis pour la formule (I), et un diisocyanate OCN-Y-NCO où Y a la signification donnée dans la formule (I) ; et éventuellement un coupleur diol ou diamine de formule H₂N-B²-NH₂ ou HO-B²-OH, où B² est tel que défini dans la formule (IX).

Suivant les proportions stoechiométriques entre les deux réactifs, diisocyanate et coupleur, on pourra avoir pour Y la formule (IX) avec d égal 0 où d égal 1 à 5.

Comme dans le cas des polyamides silicones de formule (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule : 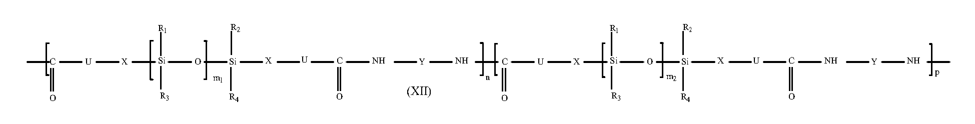 dans laquelle R¹, R², R³, R⁴, X, Y et U sont tels que définis pour la formule (VIII) et m₁, m₂, n et p sont tels que définis pour la formule (V).

On peut obtenir également des polyuréthanes ou polyurées silicones ramifiés en utilisant à la place du diisocyanate OCN-Y-NCO, un triisocyanate de formule :

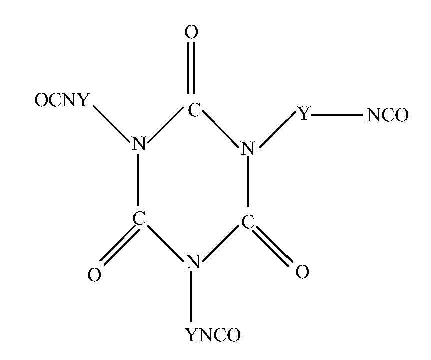

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule : 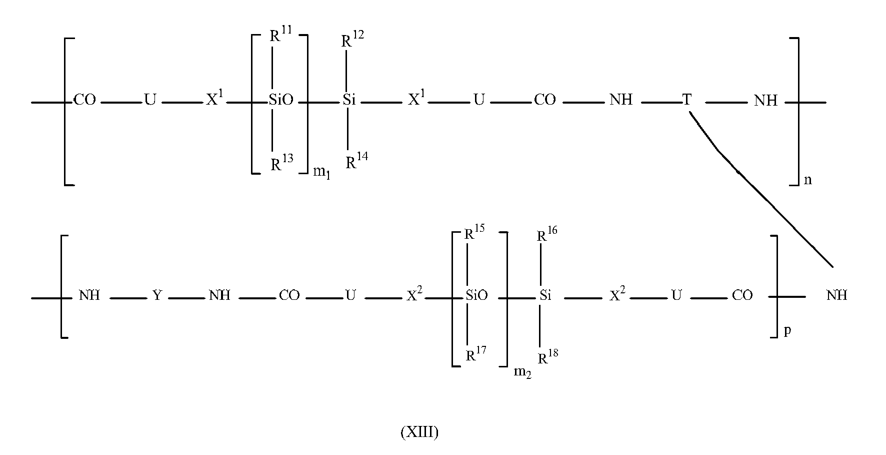 dans laquelle X¹ et X² qui sont identiques ou différents, ont la signification donnée pour X dans la formule (I), n est tel que défini dans la formule (I), Y et T sont tels que définis dans la formule (I), R¹¹ à R¹⁸ sont des groupes choisis dans le même groupe que les R¹ à R⁴, m₁ et m₂ sont des nombres situés dans la gamme allant de 1 à 1 000, et p est un nombre entier allant de 2 à 500.

Comme dans le cas des polyamides, ce copolymère peut comporter aussi des motifs polyuréthanes silicones sans ramification.

Dans ce second mode de réalisation de l'invention, les polyurées et les polyuréthanes à base de siloxanes préférés sont :
- les polymères de formule (VIII) où m est de 15 à 50 ;
- les mélanges de deux ou plusieurs polymères dans lesquels au moins un polymère a une valeur de m dans la gamme de 15 à 50 et au moins un polymère a une valeur de m dans la gamme de 30 à 50 ;
- des polymères de formule (XII) avec m₁ choisi dans la gamme de 15 à 50 et m₂ choisi dans la gamme de 30 à 500 avec la partie correspondant à m₁ représentant 1 à 99% en poids du poids total du polymère et la partie correspondant à m₂ représentant 1 à 99% en poids du poids total du polymère ;
- des mélanges de polymère de formule (VIII) combinant
   1) 80 à 99% en poids d'un polymère où n est égal à 2 à 10, en particulier 3 à 6, et
   2) 1 à 20% d'un polymère où n est dans la gamme de 5 à 500, en particulier de 6 à 100,
- des copolymères comprenant deux motifs de formule (VIII) où au moins l'un des Y contient au moins un substituant hydroxyle ;
- des polymères de formule (VIII) synthétisés avec au moins une partie d'un diacide activé (chlorure, dianhydride ou diester de diacide) au lieu du diacide ;
- des polymères de formule (VIII) où X représente - (CH₂)₃- ou -(CH₂)₁₀- ; et
- des polymères de formule (VIII) où les polymères sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Comme dans le cas des polyamides, on peut utiliser dans l'invention des copolymères de polyuréthane -ou de polyurée- silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les agents gélifiants constitués par des homopolymères ou copolymères de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales ou ramifications de la chaîne principale. Ceci peut correspondre aux cinq dispositions suivantes : 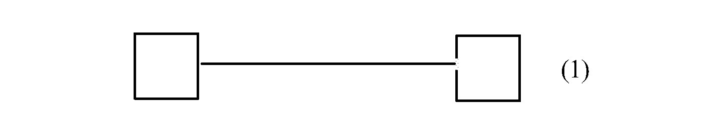 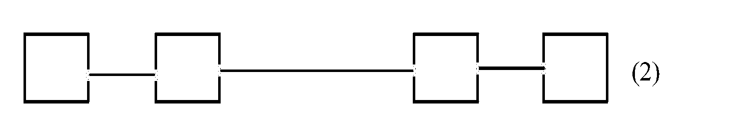 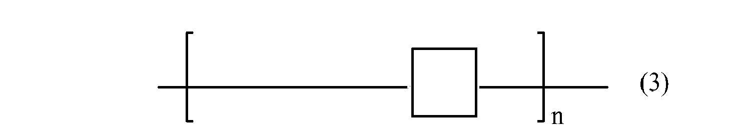 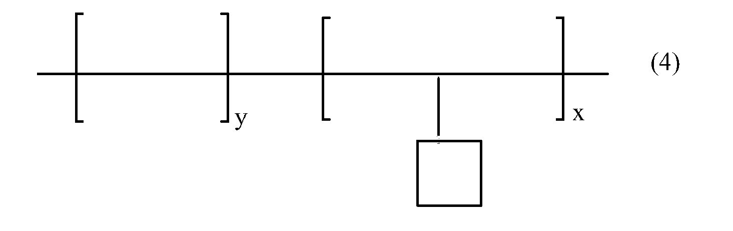 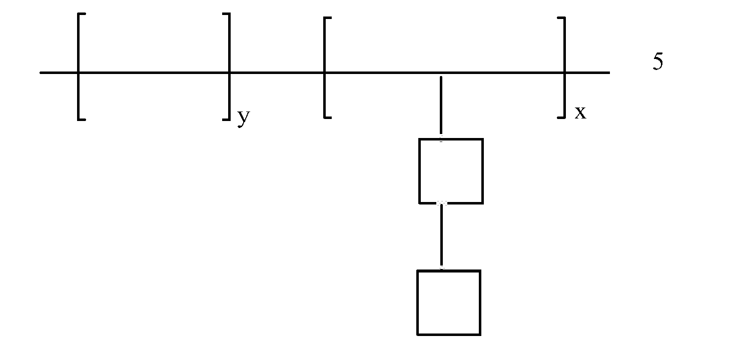 dans lesquelles, la ligne continue est la chaîne principale du polymère siloxane et les carrés représentent les groupes capables d'établir des interactions hydrogène.

Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène. Les valeurs n, x et y sont telles que le polymère présente les propriétés voulues en tant qu'agent gélifiant de phases grasses à base d'huile siliconée.

Selon l'invention, la structuration de la phase grasse liquide contenant au moins une huile siliconée est obtenue à l'aide d'un ou plusieurs des polymères mentionnés ci-dessus, en association avec des particules solides à surface hydrophobe.

A titre d'exemples de polymères utilisables, on peut citer les polyamides siliconés, obtenus conformément aux exemples 1 et 2 du document US-A-5 981 680 [5].

Les polymères et copolymères utilisés comme agents gélifiants dans la composition de l'invention ont avantageusement une température de ramollissement de 40°C à 190°C. De préférence, ils présentent une température de ramollissement allant de 50 à 140°C et mieux de 70°C à 120°C. Cette température de ramollissement est plus basse que celle des polymères structurants connus, ce qui facilite la mise en oeuvre des polymères objet de l'invention et limite les détériorations de la phase grasse liquide.

Ils présentent une bonne solubilité dans les huiles siliconées et conduisent à des compositions macroscopiquement homogènes. De préférence, ils ont une masse moléculaire moyenne de 500 à 200 000, par exemple de 1 000 à 100 000, de préférence de 2 000 à 30 000.

Selon l'invention, la composition a de préférence une dureté allant de 20 à 2 000 gf et mieux de 20 à 900 gf, notamment de 20 à 600 gf et par exemple de 150 à 450 gf. Cette dureté peut être mesurée selon une méthode de pénétration d'une sonde dans ladite composition et en particulier à l'aide d'un analyseur de texture (par exemple TA-TXT2ᵢ de chez Rhéo) équipé d'un cylindre en ébonite de 25 mm de haut et 8 mm de diamètre. La mesure de dureté est effectuée à 20°C au centre de cinq échantillons de ladite composition. Le cylindre est introduit dans chaque échantillon de composition à une pré-vitesse de 2 mm/s puis à une vitesse de 0,5 mm/s et enfin à une post-vitesse de 2 mm/s, le déplacement total étant de 1 mm. La valeur relevée de la dureté est celle du pic maximum. L'erreur de mesure est de ± 50 gf.

La dureté peut aussi être mesurée par la méthode dite du fil à couper le beurre, qui consiste à couper un bâton de rouge à lèvres de 8,1 mm de diamètre et à mesurer la dureté à 20°C, au moyen d'un dynamomètre DFGHS 2 de la société Indelco-Chatillon se déplaçant à une vitesse de 100 mm/minute. Elle est exprimée comme la force de cisaillement (exprimée en grammeforce) nécessaire pour couper un stick dans ces conditions. Selon cette méthode la dureté d'une composition en stick selon l'invention va de 30 à 300 gf, de préférence de 30 à 200 gf et par exemple de 30 à 120 gf.

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Selon l'invention, la composition sous forme de stick a le comportement d'un solide élastique déformable et souple, conférant à l'application une douceur élastique remarquable. Les compositions en stick de l'art antérieur n'ont pas cette propriété d'élasticité et de souplesse.

Le taux total de silicones amphiphiles et celui du polymère sont choisis selon la dureté de gel désirée et en fonction de l'application particulière envisagée. Les quantités respectives de polymère et de silicones amphiphiles doivent être telles qu'elles permettent l'obtention d'un stick délitable. En pratique, la quantité de polymère (en matière active) représente de 0,5 à 80% du poids total de la composition de préférence de 2% à 60% et mieux de 5 à 40%. La quantité totale de silicone(s) amphiphile(s) représente de 0,1% à 20% du poids total de la composition et mieux de 0,1% à 10%.

La composition de l'invention peut comprendre, en outre, tout ingrédient usuellement utilisé dans le domaine concerné, et notamment ceux choisis parmi les colorants solubles dans les polyols ou dans la phase grasse, les antioxydants, les huiles essentielles, les conservateurs, les parfums, les polymères liposolubles notamment hydrocarbonés tels que les polyalkylènes ou le polylaurate de vinyle, les gélifiants de phase grasse liquide, les cires, les gommes, les résines, les tensioactifs comme le phosphate de tri-oléyle, les actifs cosmétiques ou dermatologiques additionnels comme par exemple l'eau, les émollients, les hydratants, les vitamines, la lanoline liquide, les acides gras essentiels, les filtres solaires lipophiles ou solubles dans les polyols et leurs mélanges. La composition selon l'invention peut contenir également des vésicules lipidiques de type ionique et/ou non ionique. Ces ingrédients, hormis l'eau, peuvent être présents dans la composition de façon usuelle à raison de 0 à 20% du poids total de la composition et mieux de 0,1 à 10%.

Bien entendu, l'homme du métier veillera à choisir les éventuels ingrédients complémentaires et/ou leur quantité de telle manière que les propriétés avantageuses de la composition selon l'invention ne soient pas ou substantiellement pas, altérées par l'adjonction envisagée.

Au cas où la composition contiendrait une phase aqueuse, ce qui est le cas pour une émulsion simple eau-dans-huile ou huile-dans-eau ou multiple eau-dans-huile-dans-eau ou huile-dans-eau-dans-huile, cette phase aqueuse peut représenter 0,1 à 70% en poids de la composition, notamment de 0,5 à 40%, et mieux de 1 à 20%. Cette phase aqueuse peut contenir de l'eau et tout composé miscible à l'eau comme les polyols. Cette phase aqueuse peut, en outre, être gélifiée par des gélifiants appropriés. De préférence, la composition de l'invention se présente sous forme d'une phase continue grasse et plus spécialement sous forme anhydre.

Les compositions de l'invention peuvent en particulier contenir une ou plusieurs cires, par exemple de la cire de polyéthylène mais on évite l'emploi de cire si l'on veut obtenir des produits brillants. Généralement, la quantité de cire ne dépasse pas 20%, de préférence 10% ; elle représente par exemple de 3 à 5% du poids total de la composition.

La composition selon l'invention peut se présenter sous la forme d'une composition dermatologique ou de soin, éventuellement teintée, des matières kératiniques comme la peau, les lèvres et/ou les phanères, sous forme d'une composition de protection solaire ou d'hygiène corporelle notamment sous forme de produit démaquillant sous forme de stick. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, présentant éventuellement des propriétés de soin ou de traitement ; de maquillage des phanères comme les ongles, les cils en particulier sous forme d'un mascara pain, les sourcils et les cheveux notamment sous forme de crayon. En particulier, la composition de l'invention peut être un produit cosmétique contenant des actifs cosmétiques et/ou dermatologiques, comme les hydratants, les céramides, les vitamines, les filtres solaires et les agents cicatrisant.

Dans le cas des compositions de maquillage, les particules solides hydrophobes peuvent constituer le(s) pigment(s) permettant de maquiller la peau, les lèvres et/ou les phanères.

Bien entendu, la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur, de toucher et éventuellement de goût agréables.

Selon l'invention, la composition peut se présenter aussi sous la forme d'un gel rigide anhydre transparent (en l'absence de particules diffusantes comme certaines charges et pigments), et notamment de stick anhydre.

Selon l'invention, la composition peut contenir de plus une matière colorante qui peut être choisie parmi les colorants lipophiles, les colorants hydrophiles, et leurs mélanges.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine, le rocou. Ils peuvent représenter de 0 à 20% du poids de la composition et mieux de 0,1 à 6% (si présents).

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le(s) huile(s), les particules, la ou les silicones amphiphiles, si nécessaire les matières colorantes et les additifs puis à mélanger le tout jusqu'à l'obtention d'une solution homogène à l'oeil nu. Le mélange homogène obtenu peut alors être coulé dans un moule approprié comme un moule de rouge à lèvres ou directement dans les articles de conditionnement (boîtier ou coupelle notamment).

L'invention a encore pour objet un procédé cosmétique de soin, de maquillage ou de traitement des matières kératiniques des êtres humains et notamment de la peau, des lèvres et des phanères, comprenant l'application sur les matières kératiniques de la composition notamment cosmétique telle que définie ci-dessus.

L'invention a aussi pour objet l'utilisation d'une quantité suffisante d'au moins un polymère (homopolymère ou copolymère) de masse moléculaire moyenne en poids allant de 500 à 500 000 et mieux de 2 000 à 30 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, contenant une phase grasse continue liquide comprenant au moins une huile siliconée, pour structurer ladite composition sous forme d'un solide autoporté de dureté allant de 20 à 2 000 gf et de préférence de 20 à 900 gf et mieux de 20 à 600 gf.

L'invention a encore pour objet l'utilisation d'une phase grasse liquide continue comprenant au moins une huile siliconée, structurée essentiellement par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, rigide autoportée, brillante et/ou non migrante.

L'invention a encore pour objet l'utilisation d'une quantité suffisante d'au moins un polymère (homopolymère ou copolymère) de masse moléculaire moyenne en poids de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, contenant une phase grasse continue liquide comprenant au moins une huile siliconée, pour structurer ladite composition sous forme d'un solide autoporté.

L'invention concerne encore l'utilisation d'une phase grasse liquide continue, comprenant au moins une huile siliconée, structurée essentiellement par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, comme agent pour limiter la migration de ladite composition.

L'invention a encore pour objet un procédé cosmétique pour limiter la migration d'une composition cosmétique contenant une phase grasse liquide comprenant au moins une huile siliconée, consistant à structurer ladite phase grasse par une quantité suffisante d'au moins un polymère (homopolymère ou copolymère) de masse moléculaire moyenne en poids allant de 500 à 500 000, comportant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile.

L'invention a encore pour objet un stick de maquillage de la peau, des lèvres et/ou des phanères, et en particulier des lèvres, contenant des particules solides hydrophiles constituées par au moins un pigment en quantité suffisante pour maquiller la peau, les lèvres et/ou les phanères, au moins une silicone amphiphile, et une phase grasse continue liquide comprenant au moins une huile siliconée, structurée par au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, uréthane ou carbamate, thiocarbamate, urée, thiourée, oxamido, guanidino, biguanidino et leurs combinaisons, à condition qu'au moins un des groupes soit différent d'un groupe ester,
le pigment, la phase grasse, la silicone amphiphile et le polymère formant un milieu physiologiquement acceptable.

L'invention est illustrée plus en détail dans les exemples suivants de formulation de maquillage contenant un polyamide siliconé, des pigments hydrophiles (oxydes de fer et oxyde de titane non traités) et une silicone amphiphile constituée par du polydiméthylsiloxane (PDMS) oxyéthyléné éventuellement mélangé à du PDMS oxyéthyléné/oxypropyléné. Dans l'un des exemples, on ajoute de plus un composé amphiphile non siliconé, constitué par l'acide poly(12-hydroxystéarique). Les quantités sont données en % massique. Les composés chimiques sont donnés principalement en nom CTFA ("International Cosmetic Ingredient Dictionary"). Les viscosités indiquées sont mesurées à 25°C, à la pression atmosphérique.

### Exemple 1 : Rouge à lèvres

| COMPOSITION | |
|---|---|
| Acide poly(12-hydroxystéarique) Solperse 21 000) | 2% |
| PDMS oxyéthyléné/oxypropyléné en α-ω dans le cyclopentasiloxane D5 (Abil EM 90 de ches Golsdschmidt) | 3% |
| Phényltriméthicone (DC 556 de chez Dow Corning, de 20 cSt) | 40% |
| Isoparaffine hydrogénée (Parleam^{®} de chez Nippon Oil Fats) | 18% |
| Pigments (oxydes de fer rouge, jaune et oxyde de titane) | 10% |
| Cire de polyéthylène (Performalen^{®} 500 de chez Petrolite) | 12% |
| Polyamide siliconé de l'exemple 2 du brevet US 5 981 680 | 15% |
| Conservateur qs | |
| Parfum qs | |

Les pigments présentent les indices de couleur (CI) suivants :
oxyde de fer rouge CI : 77494 (95/5)
oxyde de fer jaune CI : 77492 (95/5)
oxyde de titane CI : 77891 (95/5)
95/5 signifie que l'on a 95% en poids d'oxyde et 5% en poids d'enrobage.

Ce rouge à lèvres est obtenu en chauffant la cire et le polymère dans une partie de la phase grasse liquide, contenant le Parleam, et la phényltriméthicone. Parallèlement, on mélange les pigments, le SOLPERSE, l'Abil EM 90 dans l'autre partie des huiles à température ambiante puis on les passe au broyeur tricylindre. On ajoute ce broyat au mélange fondu de cire et d'huiles de silicone, puis on homogénéise l'ensemble. On ajoute le conservateur et le parfum toujours sous agitation, puis on coule le mélange dans un moule approprié.

Après refroidissement à la température ambiante, on obtient un gel mou glissant bien sur les lèvres et formant sur celles-ci un dépôt épais, très couvrant, plutôt mat et onctueux, ne laissant pas apparaître les lèvres.

Le produit ainsi obtenu présente des propriétés de tenue en particulier de la couleur, de glissant et de non gras.

Le polyamide siliconé utilisé dans cet exemple comprend 20 motifs [Si(CH₃)₂-O]. Si l'on utilise un polymère dont le nombre de motifs [Si(CH₃)₂-O] est plus élevé, on obtient un gel plus dur et brillant qui est transparent à l'état fondu.

### Exemple 2 : Fond de teint anhydre

| COMPOSITION | |
|---|---|
| PDMS (10 cSt) | qsp 100% |
| Phényltriméthicone (DC 556) | 12% |
| PDMS oxyéthyléné (500 cSt) | 3% |
| PDMS oxyéthyléné/oxypropyléné en α-ω dans le cyclopentasiloxane | 2% |
| D5 (ABIL EM 97 de chez Goldschmidt) PDMS : D5(-85/15) Pigments (oxydes de fer rouge, jaune et oxyde de titane) | 10% |
| Cire de polyéthylène (Performalen^{®} 500) | 15% |
| Polyamide siliconé de l'exemple 2 du brevet US 5 981 680 | 12% |
| Silice traitée hydrophobe (traitement triméthysiloxyl) | 3% |
| Isononanoate d'isononyle | 10% |
| Conservateur qs | |
| Parfum qs | |

Ce fond de teint est préparé comme dans l'exemple 1, la silice étant introduite en même temps que la phényltriméthicone dans le broyat pigmentaire et l'isononanoate d'isononyle étant introduit dans le mélanges de cire et d'huiles de silicone.

Il présente des propriétés de non gras, de glissant, de matité et de bonne tenue dans le temps en particulier de la couleur.

### Exemple 3 : Fond de teint

| COMPOSITION | |
|---|---|
| PDMS (10cSt) | qsp 100% |
| Phényltriméthicone (DC 556) | 12% |
| PDMS oxyéthyléné (500 cSt) | 3% |
| PDMS oxyéthyléné/oxypropyléné en α-ω dans le cyclopentasiloxane D5 (ABIL EM 97 de chez Goldschmidt) PDMS : D5-85/15 | 2% |
| Pigments (oxydes de fer rouge, jaune et nanooxyde de titane traité par l'alumine puis PDMS) | 10% |
| Cire de polyéthylène (Performalen^{®} 500) | 15% |
| Polyamide siliconé de l'exemple 2 du brevet US 5 981 680 | 12% |
| Silice traitée hydrophobe (traitement triméthysiloxyl) | 3% |
| Isononanoate d'isononyle | 10% |
| Conservateur qs | |
| Parfum qs | |

Ce fond de teint est préparé en suivant le même mode opératoire que dans l'exemple 2. Ces propriétés cosmétiques sont identiques à celle du fond de teint de l'exemple 2.

### Exemple 4 : Fond de teint

La composition de ce fond de teint est identique à celle de l'exemple 3 sauf que l'on utilise comme pigments, des pigments traités hydrophobes (oxyde de fer rouge, jaune et oxyde de titane, traités par du perfluoroalkylphosphate), au lieu des pigments (oxydes de fer rouge, jaune et nanooxyde de titane traité par l'alumine puis PDMS).

On suit le même mode opératoire que dans l'exemple 2 et on obtient un fond de teint ayant des propriétés identiques à celles du fond de teint de l'exemple 2.

### REFERENCES CITEES

**[1]** EP-A-1 068 856
**[2]** US-A-5,874,069
**[3]** US-A-5, 919, 441
**[4]** US-A-6,051,216
**[5]** US-A-5, 981, 680
**[6]** US-A-5 412 004,
**[7]** EP-A- 1 048 686,
**[8]** WO-A-02/17870
**[9]** WO-A-02/17871

## Revendications

1. Composition comprenant une phase grasse liquide comprenant au moins une huile siliconée, structurée par l'association :
1) d'au moins un agent gélifiant constitué par un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
2) de particules solides, et
3) d'au moins une silicone amphiphile,
la phase grasse liquide, le polymère, les particules solides et la silicone amphiphile formant un milieu physiologiquement acceptable.

2. Composition selon la revendication 1, dans laquelle les particules solides sont choisies parmi les charges et les pigments.

3. Composition selon la revendication 2, dans laquelle les particules solides sont des particules hydrophiles, sous forme de poudres ou de fibres.

4. Composition selon la revendication 3, dans laquelle les particules hydrophiles sont des pigments choisis parmi les oxydes de zinc, les oxydes de fer et les oxydes de titane.

5. Composition selon l'une quelconque des revendications 1 à 4, dans laquelle la silicone amphiphile est choisie parmi les silicones du type huile sans activité gélifiante.

6. Composition selon la revendication 5, dans laquelle la silicone est choisie parmi les diméthicone copolyols, les alkylméthicone copolyols, les silicones polyglycérolées, les silicones à groupes latéraux perfluorés et à groupes latéraux glycérolés, les silicones à groupes latéraux polyoxyéthylénés, polyoxypropylénés et fluorés, les copolymères à bloc silicone et à bloc hydrophile autre que polyéther, et les polysaccharides greffés silicone.

7. Composition selon l'une quelconque des revendications 1 à 4, dans laquelle la silicone amphiphile est une résine silicone, au moins partiellement réticulée.

8. Composition selon la revendication 7, dans laquelle la résine silicone est une résine silicone à groupe alkylpolyéther, ou une résine silicone réticulée partiellement par des α,ω-diènes, possédant des chaînes latérales polyoxyéthylénés et polyoxypropylénées hydrophiles et des chaînes latérales alkyle hydrophobes.

9. Composition selon l'une quelconque des revendications 1 à 4, dans laquelle la (les) silicone (s) amphiphile (s) sont choisies parmi les polydiméthylsiloxanes oxyéthylénés et les polydiméthylsiloxanes oxyéthylénés/oxypropylénés.

10. Composition selon l'une quelconque des revendications 1 à 9, dans laquelle le polymère utilisé comme agent gélifiant comprend au moins un motif répondant à la formule : 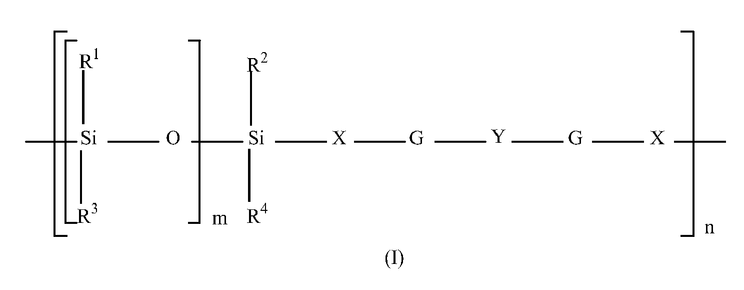 dans laquelle :
1) R¹, R², R³ et R⁴, identiques ou différents, représentent un groupe choisi parmi :
- les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C₆ à C₁₀, éventuellement substitués par un ou plusieurs groupes alkyle en C₁ à C₄,
- les chaînes polyorganosiloxanes contenant ou non un ou plusieurs atomes d'oxygène, de soufre et/ou d'azote ;
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote ;
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 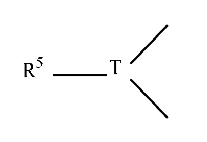 dans laquelle
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
- R⁵ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 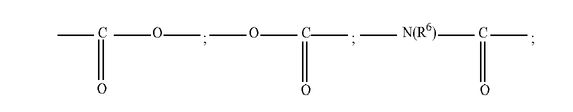 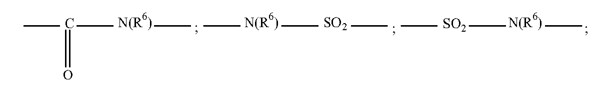 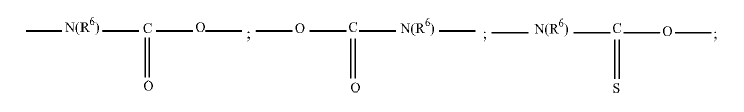 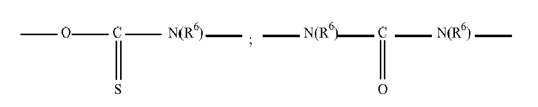 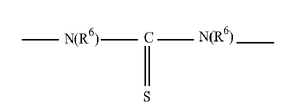 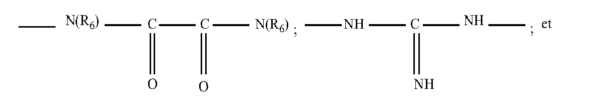 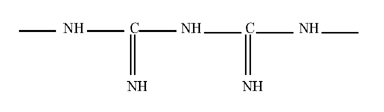 où R⁶ représente un atome d'hydrogène ou un groupe alkyle, linéaire ou ramifié, en C₁ à C₂₀, à condition qu'au moins 50% des R⁶ du polymère représente un atome d'hydrogène et qu'au moins deux des groupes G du polymère soient un autre groupe que : 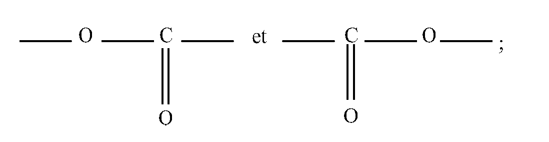
6) n est un nombre entier allant de 2 à 500, de préférence de 2 à 200, et m est un nombre entier allant de 1 à 1000, de préférence de 1 à 700 et mieux encore de 6 à 200.

11. Composition selon la revendication 10, dans laquelle Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆, et
g) les chaînes polyorganosiloxane de formule : 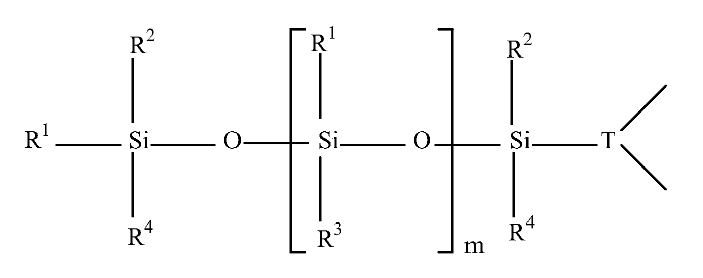
dans laquelle R¹, R², R³, R⁴, T et m sont tels que définis ci-dessus.

12. Composition selon l'une quelconque des revendications 1 à 9, dans laquelle le polymère utilisé comme agent gélifiant comprend au moins un motif répondant à la formule (II) : 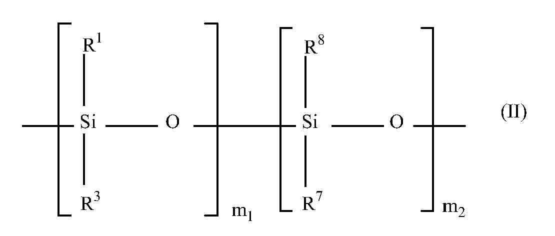 dans laquelle
- R¹ et R³, identiques ou différents, sont tels que définis ci-dessus pour la formule (I) dans la revendication 10,
- R⁷ représente un groupe tel que défini ci-dessus pour R¹ et R³, ou représente le groupe de formule -X-G-R⁹ dans laquelle X et G sont tels que définis ci-dessus pour la formule (I) dans la revendication 10, et R⁹ représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
- R⁸ représente le groupe de formule -X-G-R⁹ dans laquelle X, G et R⁹ sont tels que définis ci-dessus,
- m₁ est un nombre entier allant de 1 à 998, et
- m₂ est un nombre entier allant de 2 à 500.

13. Composition selon la revendication 10, dans laquelle le polymère comprend au moins un motif de formule (III) ou (IV) : 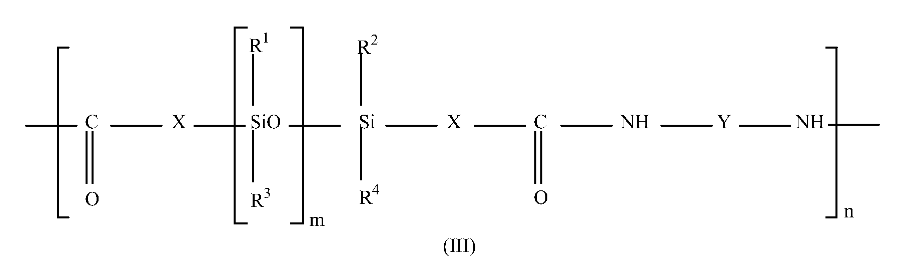 ou 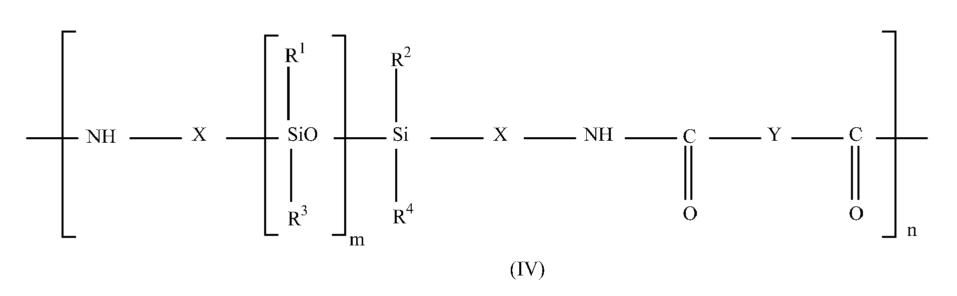 dans lesquelles R¹, R², R³, R⁴, X, Y, m et n sont tels que définis dans la revendication 10.

14. Composition selon l'une quelconque des revendications 10 et 13, dans laquelle X et/ou Y représentent un groupe alkylène contenant dans sa partie alkylène au moins l'un des éléments suivants :
1°) 1 à 5 groupes amides, urée ou carbamate,
2°) un groupe cycloalkyle en C₅ ou C₆, et
3°) un groupe phénylène éventuellement substitué par 1 à 3 groupes alkyles identiques ou différents en C₁ à C₃, et/ou substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

15. Composition selon l'une quelconque des revendications 10 à 13, dans laquelle Y représente : 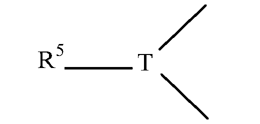 où R⁵ représente une chaîne polyorganosiloxane, et T représente un groupe de formule : 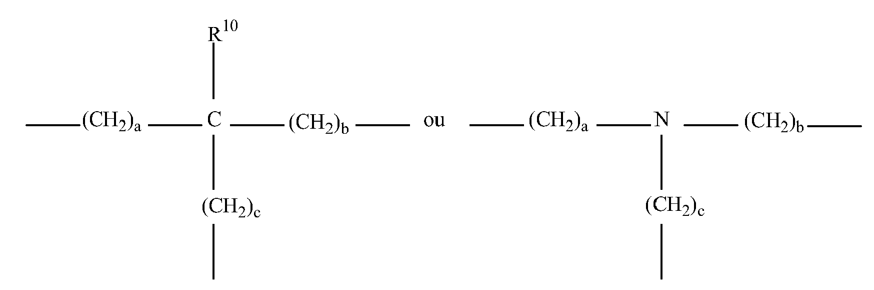 dans lesquelles a, b et c sont indépendamment des nombres entiers allant de 1 à 10, et R¹⁰ est un atome d'hydrogène ou un groupe tel que ceux définis pour R¹, R², R³ et R⁴, dans la revendication 10.

16. Composition selon l'une quelconque des revendications 10 à 13, dans laquelle R¹, R², R³ et R⁴ représentent, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

17. Composition selon la revendication 1, dans laquelle le polymère utilisé comme agent gélifiant comprend au moins un motif de formule : 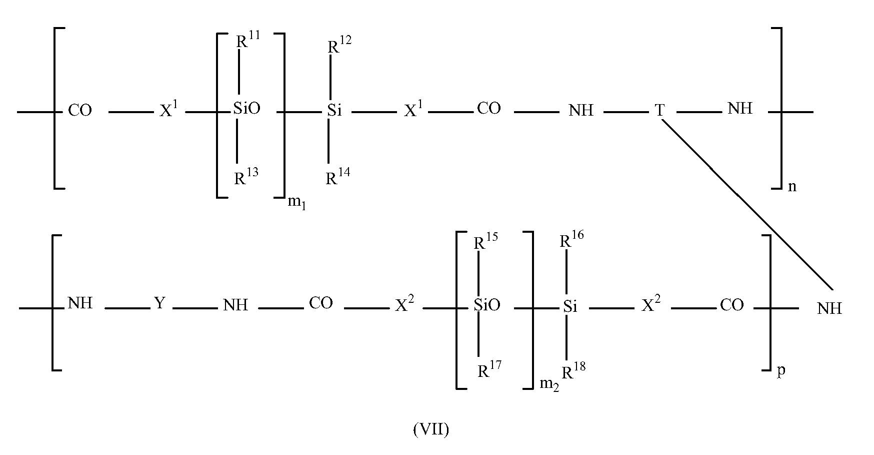 dans laquelle X¹ et X² qui sont identiques ou différents, ont la signification donnée pour X dans la revendication 10, n, Y et T sont tels que définis dans la revendication 10, R¹¹ à R¹⁸ sont des groupes choisis dans le même groupe que les R¹ à R⁴ de la revendication 10, m₁ et m₂ sont des nombres situés dans la gamme allant de 1 à 1 000, et p est un nombre entier allant de 2 à 500.

18. Composition selon la revendication 17, dans laquelle :
- p est dans la gamme de 1 à 25, mieux encore de 1 à 7,
- R¹¹ à R¹⁸ sont des groupes méthyle,
- T répond à l'une des formules suivantes : 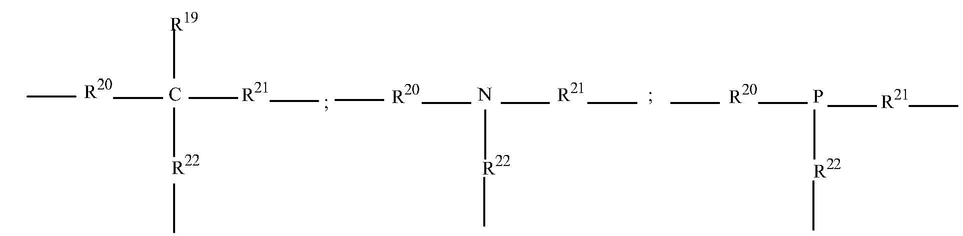 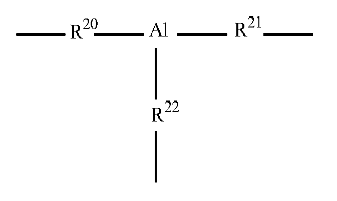
dans lesquelles R¹⁹ est un atome d'hydrogène ou un groupe choisi parmi les groupes définis pour R¹ à R⁴, et R²⁰ R²¹ et R²² sont indépendamment des groupes alkylène, linéaires ou ramifiés, de préférence encore, à la formule : 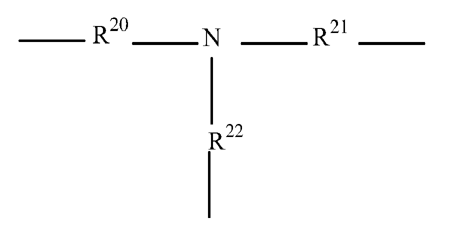 en particulier avec R²⁰, R²¹ et R²² représentant -CH₂-CH₂-,
- m₁ et m₂ sont dans la gamme de 15 à 500, et mieux encore de 15 à 45,
- X¹ et X² représentent -(CH₂)₁₀-, et
- Y représente -CH₂-.

19. Composition selon l'une quelconque des revendications 1 à 9, dans laquelle le polymère comprend au moins un motif répondant à la formule suivante : 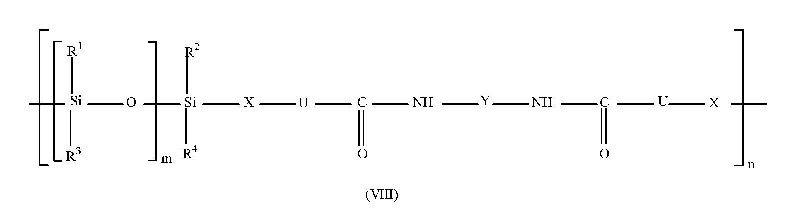 dans laquelle les R¹, R², R³, R⁴, X, Y, m et n ont les significations données ci-dessus pour la formule (I) dans la revendication 10, et U représente -O- ou -NH-, ou
Y représente un groupe cycloaliphatique ou aromatique en C₅ à C₁₂ pouvant être substitué par un groupe alkyle en C₁ à C₁₅ ou un groupe aryle en C₅ à C₁₀, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane, ou Y représente un radical alkylène en C₁ à C₄₀, linéaire ou ramifié, ou un radical cycloalkylène en C₄ à C₁₂, ou
Y représente une séquence polyuréthane ou polyurée correspondant à la condensation de plusieurs molécules de diisocyanate avec une ou plusieurs molécules de coupleurs du type diol ou diamine, répondant à la formule : 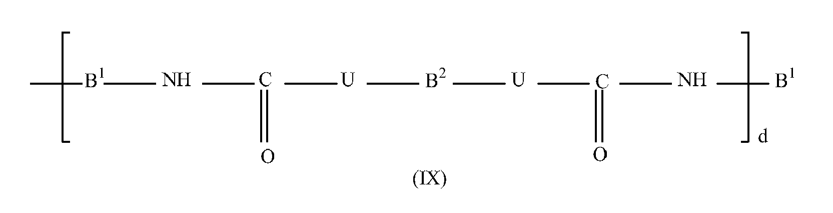 dans laquelle B¹ est un groupe choisi parmi les groupes donnés ci-dessus pour Y, U est -O- ou -NH-, et B² est choisi parmi :
• les groupes alkylène en C₁ à C₄₀, linéaires ou ramifiés, qui peuvent porter éventuellement un groupe ionisable tel qu'un groupe acide carboxylique ou sulfonique, ou un groupe amine tertiaire neutralisable ou quaternisable,
• les groupes cycloalkylène en C₅ à C₁₂, éventuellement porteurs de substituants alkyle, par exemple un à trois groupes méthyle ou éthyle, ou alkylène, par exemple le radical du diol : cyclohexane diméthanol,
• les groupes phénylène pouvant éventuellement être porteurs de substituants alkyles en C₁ à C₃, et
• les groupes de formule : 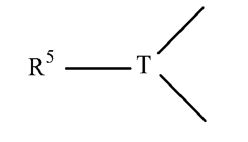
dans laquelle T est un radical trivalent hydrocarboné pouvant contenir un ou plusieurs hétéroatomes tels que l'oxygène, le soufre et l'azote et R⁵ est une chaîne polyorganosiloxane ou une chaîne alkyle en C₁ à C₅₀, linéaire ou ramifiée.

20. Composition selon l'une quelconque des revendications 1 à 9, dans laquelle le polymère comprend au moins un motif de formule : 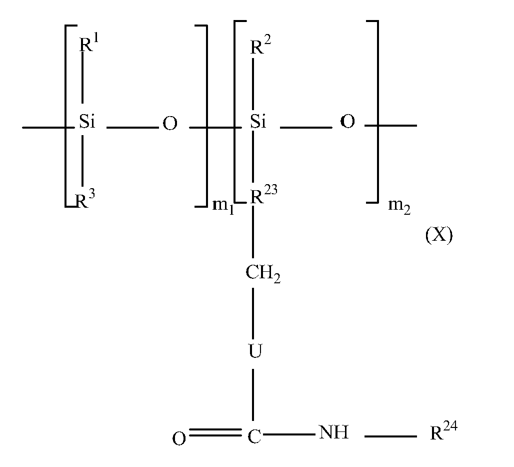 dans laquelle R¹, R², R³, m₁ et m₂ ont les significations données ci-dessus pour la formule (I),
- U représente O ou NH,
- R²³ représente un groupe alkylène en C₁ à C₄₀, comportant éventuellement un ou plusieurs hétéroatomes choisis parmi O et N, ou un groupe phénylène, et
- R²⁴ est choisi parmi les groupes alkyle en C₁ à C₅₀, linéaires, ramifiés ou cycliques, saturés ou insaturés, et les groupes phényle éventuellement substitués par un à trois groupes alkyles en C₁ à C₃.

21. Composition selon l'une quelconque des revendications 1 à 9, dans laquelle le polymère utilisé comme agent gélifiant comprend au moins un motif de formule : 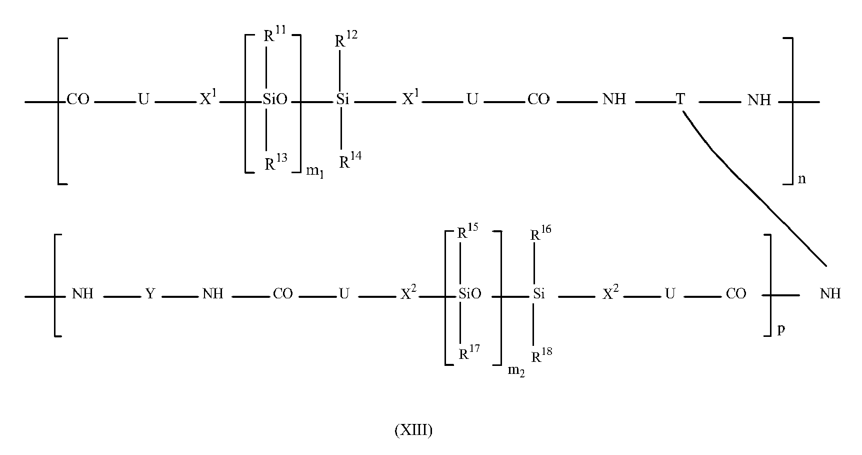 dans laquelle X¹ et X² qui sont identiques ou différents, ont la signification donnée pour X dans la revendication 10, n, Y et T sont tels que définis dans la revendication 10, R¹¹ à R¹⁸ sont des groupes choisis dans le même groupe que les R¹ à R⁴ de la revendication 10, m₁ et m₂ sont des nombres situés dans la gamme allant de 1 à 1 000, et p est un nombre entier allant de 2 à 500.

22. Composition selon l'une quelconque des revendications 10 à 21, dans laquelle le polymère utilisé comme agent gélifiant comprend de plus un motif hydrocarboné comportant deux groupes capables d'établir des interactions hydrogène choisis parmi les groupes ester, amide, sulfonamide, carbamate, thiocarbamate, urée, thiourée et leurs combinaisons.

23. Composition selon la revendication 22, dans laquelle le copolymère est un copolymère bloc, un copolymère séquencé ou un copolymère greffé.

24. Composition selon l'une quelconque des revendications précédentes, dans laquelle le polymère représente de 0,5 à 80% du poids total de la composition, de préférence de 2 à 60% et mieux de 5 à 40%, les particules solides représentent de 0,1 à 90%, de préférence de 1 à 70% et mieux de 2 à 50%, du poids total de la composition, et la silicone amphiphile représente de 0,1 à 20% et mieux de 0,1 à 10% du poids total de la composition.

25. Composition selon l'une quelconque des revendications précédentes, dans laquelle la phase grasse liquide contient au moins 40% et mieux encore au moins 50% en poids d'huile siliconée.

26. Composition selon la revendication 25, dans laquelle la phase grasse liquide contient de plus une huile non siliconée.

27. Composition selon l'une quelconque des revendications précédentes, dans laquelle la phase grasse liquide représente de 5 à 99% du poids total de la composition et mieux de 20 à 75%.

28. Composition selon l'une quelconque des revendications précédentes, **caractérisée en ce qu'**elle constitue une composition de soin et/ou de traitement et/ou de maquillage des matières kératiniques.

29. Composition selon l'une des revendications précédentes, **caractérisée en ce qu'**elle contient, en outre, au moins un actif cosmétique ou dermatologique.

30. Composition selon l'une quelconque des revendications précédentes, **caractérisée en ce qu'**elle contient au moins un additif choisi parmi l'eau, les antioxydants, les huiles essentielles, les conservateurs, les parfums, les polymères liposolubles notamment hydrocarbonés tels que les polyalkylènes ou le polylaurate de vinyle, les gélifiants de phase grasse liquide, les cires, les gommes, les résines, les tensioactifs comme le phosphate de tri-oléyle, les actifs cosmétiques ou dermatologiques additionnels choisis dans le groupe constitué par les émollients, les hydratants, les vitamines, la lanoline liquide, les acides gras essentiels, les filtres solaires lipophiles ou solubles dans les polyols et leurs mélanges

31. Composition selon l'une quelconque des revendications précédentes, **caractérisée en ce qu'**elle se présente sous la forme d'un gel rigide anhydre transparent, et notamment de stick anhydre transparent.

32. Composition solide structurée de maquillage de la peau, des lèvres et/ou des phanères, contenant des particules solides hydrophiles constituées par au moins un pigment en quantité suffisante pour maquiller la peau, les lèvres et/ou les phanères, au moins une silicone amphiphile et une phase grasse continue liquide comprenant au moins une huile siliconée, structurée par au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
ladite composition se présentant sous forme d'un solide, le pigment, la phase grasse liquide, la silicone amphiphile et le polymère formant un milieu physiologiquement acceptable.

33. Composition selon la revendication 28, **caractérisée en ce qu'**elle est autoportée.

34. Composition structurée de rouge à lèvres, contenant des particules solides hydrophiles, constituées par au moins un pigment en quantité suffisante pour maquiller les lèvres, au moins une silicone amphiphile et une phase grasse continue liquide comprenant au moins une huile siliconée, structurée par au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
ladite composition se présentant sous forme d'un solide, le pigment, la phase grasse liquide, la silicone amphiphile et le polymère formant un milieu physiologiquement acceptable.

35. Composition selon la revendication 34, dans laquelle le polymère comprend au moins un motif répondant à l'une des formules suivantes : formule (I) de la revendication 10, formule (II) de la revendication 12, formules (III) et (IV) de la revendication 13, formule (VII) de la revendication 17, formule (VIII) de la revendication 19, formule (X) de la revendication 20 et formule (XIII) de la revendication 21.

36. Composition selon l'une quelconque des revendications 1 à 33, **caractérisé en ce qu'**elle se présente sous forme de mascara pain, d'eye-liner, de fond de teint, de rouge à lèvres, de blush, de produit déodorant ou démaquillant, de produit de maquillage du corps, de fard à paupières ou à joues, de produit anti-cerne.

37. Stick de maquillage de la peau, des lèvres et/ou des phanères, et en particulier des lèvres, contenant des particules solides hydrophiles constituées par au moins un pigment en quantité suffisante pour maquiller la peau, les lèvres et/ou les phanères, au moins une silicone amphiphile et une phase grasse continue liquide comprenant au moins une huile siliconée, structurée par au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le pigment, la phase grasse, la silicone amphiphile et le polymère formant un milieu physiologiquement acceptable.

38. Procédé cosmétique de soin, de maquillage ou de traitement des matières kératiniques des êtres humains, comprenant l'application sur les matières kératiniques d'une composition cosmétique conforme à l'une des revendications précédentes.

39. Utilisation d'une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, contenant une phase grasse continue liquide comprenant au moins une huile siliconée, pour structurer ladite composition sous forme d'un solide autoporté de dureté allant de 20 à 2 000 gf et de préférence de 20 à 900 gf et mieux de 20 à 600 gf.

40. Utilisation selon la revendication précédente, **caractérisée en ce que** le polymère comprend au moins un motif répondant à l'une des formules suivantes : formule (I) de la revendication 10, formule (II) de la revendication 12, formules (III) et (IV) de la revendication 13, formule (VII) de la revendication 17, formule (VIII) de la revendication 19, formule (X) de la revendication 20 et formule (XIII) de la revendication 21.

41. Utilisation d'une phase grasse liquide continue comprenant au moins une huile siliconée, structurée essentiellement par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, rigide autoportée, brillante et/ou non migrante.

42. Utilisation selon la revendication précédente, dans laquelle le polymère comprend au moins un motif répondant à l'une des formules suivantes : formule (I) de la revendication 10, formule (II) de la revendication 12, formules (III) et (IV) de la revendication 13, formule (VII) de la revendication 17, formule (VIII) de la revendication 19, formule (X) de la revendication 20 et formule (XIII) de la revendication 21.

43. Utilisation d'une quantité suffisante d'au moins un polymère (homopolymère ou copolymère) de masse moléculaire moyenne en poids de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, contenant une phase grasse continue liquide comprenant au moins une huile siliconée, pour structurer ladite composition sous forme d'un solide autoporté.

44. Utilisation d'une phase grasse liquide continue, comprenant au moins une huile siliconée, structurée essentiellement par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile,
dans une composition cosmétique ou pour la fabrication d'une composition physiologiquement acceptable, comme agent pour limiter la migration de ladite composition.

45. Utilisation selon l'une quelconque des revendications 39 à 44, dans laquelle la composition a une dureté de 20 à 2 000 gf, de préférence de 20 à 900 gf et mieux de 20 à 600 gf.

46. Procédé cosmétique pour limiter la migration d'une composition cosmétique contenant une phase grasse liquide comprenant au moins une huile siliconée, consistant à structurer ladite phase grasse par une quantité suffisante d'au moins un polymère (homopolymère ou copolymère) de masse moléculaire moyenne en poids allant de 500 à 500 000, comportant au moins un motif comprenant :
- au moins un groupe polyorganosiloxane, constitué de 1 à 1000 unités organosiloxanes dans la chaîne du motif ou sous forme de greffon, et
- au moins deux groupes capables d'établir des interactions hydrogène choisis parmi les groupes ester, amide, sulfonamide, carbamate, thiocarbamate, urée, thiourée, oxamido, guanidino, biguanidino et leurs combinaisons, à condition qu'au moins un des groupes soit différent d'un groupe ester,
le polymère étant solide à la température ambiante et soluble dans la phase grasse liquide à une température de 25 à 250°C,
de particules solides hydrophiles, et
d'au moins une silicone amphiphile.
